# EUROPEAN PATENT APPLICATION

(11) **EP 4 806 992 A1**
(43) Date of publication of application: **16.09.2026**
(21) Application number: 24889173.1
(22) Date of filing: 08.11.2024
(51) Int. Cl.: C12N 9/12, C12N 15/74, A61K 48/00, A61K 38/45, A61P 35/00, C12Q 1/6897, A61K 35/00

(54) **DNA CONSTRUCT FOR EXPRESSING SIGNALING SUBSTANCE FOR CANCER TREATMENT, AND CANCER TREATMENT METHOD USING SAME**

(30) Priority: 09.11.2023 KR 20230154107
(71) Applicant: Industry Foundation of Chonnam National University, Gwangju 61186 (KR); CNCure Co., Ltd., Jeollanam-do 58128 (KR)
(72) Inventor: MIN, Jung-Joon, Gwangju 61461 (KR); YOU, Sung-Hwan, Gwangju 61270 (KR); HONG, Yeongjin, Gwangju 61682 (KR)
(74) Representative: V.O.
(86) International application number: PCT/KR2024/017622
(87) International publication number: WO 2025/100987

(57) **Abstract**

Cyclic dinucleotides, which are a type of signaling substance for cancer treatment, are secondary messengers of intracellular events initiated by GPCR activation and act as a stimulator of interferon genes (STING), and thus can have a significant effect on tumor suppression. Meanwhile, when cancer occurs in a subject, angiogenesis and cell growth proceed at a very high rate in the body, and thus an oxygen-deficient environment is created due to incomplete angiogenesis in cancer tissues, which may be very suitable for the proliferation of anaerobic bacteria such as Salmonella sp. strains or E. coli.

Therefore, the present invention relates to a DNA construct into which is introduced a gene for an enzyme that synthesizes a signaling substance for cancer treatment, e.g., a cyclic dinucleotide, or to a strain transformed with a vector comprising the DNA construct. The DNA construct or the strain transformed with a vector comprising the DNA construct, according to the present invention, targets cancer in a subject and then secretes C-di-AMP or C-di-GMP synthase in the surrounding environment of the cancer, and thus can very effectively prevent or treat cancer and, at the same time, can diagnose cancer in real time.

## Description

### [Technical Field]

The present invention relates to a DNA construct for expressing a signaling substance for cancer treatment, such as a cyclic dinucleotide, a strain into which a recombinant vector comprising the DNA construct is introduced, and a cancer treatment method using the same.

### [Background Art]

Cyclic dinucleotides, which are a type of signaling substance for cancer treatment, are known to be secondary messengers of intracellular events initiated by activation of G-protein-coupled receptors (GPCRs), and to act as a stimulator of interferon genes (STING) capable of inducing type I interferons. The stimulator of interferon genes is a signaling molecule encoded by the TMEM173 gene in humans. STING is a protein having 379 amino acids composed of multiple transmembrane domains. The STING protein is expressed in various endothelial and epithelial cell types as well as in hematopoietic lineages that may include or exclude dendritic cells (DCs), including T cells, plasmacytoid dendritic cells (pDCs), and macrophages. STING is associated with the endoplasmic reticulum (ER) within cells and plays an important role in regulating the transcription of numerous host defense genes, including type I interferons (IFNs) and pro-inflammatory cytokines. STING is activated upon recognition of abnormal DNA species or cyclic dinucleotides (CDNs) in the cytoplasm of cells. Cytosolic DNA species may activate STING signaling after binding to cyclic GMP-AMP synthase (cGAS). Binding of cytosolic DNA to cGAS catalyzes the production of a CDN type known as cGAMP (cyclic GMP-AMP), which contains one 2',5'-phosphodiester linkage and a standard 3',5' linkage (c[G(2,5')pA(3,5')p]). Binding of cGAMP and other bacterial CDNs induces a conformational change in the STING protein and promotes binding of TANK-binding kinase 1 (TBK1). The STING-TBK1 complex further translocates to the perinuclear region of the cell, thereby transporting TBK1 to endolysosomal compartments where transcription factors such as interferon regulatory factor 3 (IRF3) are phosphorylated. Similarly, STAT6 and nuclear factor-κB (NF-κB) are also activated downstream of STING activation. These transcription factors then translocate to the nucleus to initiate transcription of innate immune genes and production of type I IFNs and other cytokines. Thereafter, STING is rapidly degraded, thereby avoiding problems associated with sustained cytokine production (Nature Reviews Immunol, 2015, 15:760-770; Cell Reports, 2015, 11:1018-1030). Studies in mice have shown that type I IFN signaling plays an important role in tumor-initiating T cell priming and tumor control (J. Exp. Med. 2011, 208, 1989-2003). Mice lacking IFN-α/β receptors in DCs failed to reject immunogenic tumors, and CD8α+ DCs in such mice are defective in antigen cross-presentation to CD8+ T cells. In addition, transcriptional profiling analysis of melanoma patients revealed that tumors containing infiltrating activated T cells are characterized by a type I IFN transcriptional signature (Cancer Res. 2009, 69:3077-3085). Therefore, activation of STING is expected to have a significant effect on tumor suppression.

Meanwhile, when cancer occurs in a subject, angiogenesis and cell growth proceed at a very rapid rate in the body, and thus, due to incomplete angiogenesis, an oxygen-deficient environment is formed within cancer tissues, which may be highly suitable for the proliferation of anaerobic bacteria such as *Salmonella* sp. strains or *E. coli*. Accordingly, efforts have been continuously made to develop cancer treatment methods using bacteria capable of targeting cancer, such as *Salmonella* genus strains and *Clostridium* genus strains. However, most anaerobic bacteria may cause diseases in animal subjects including humans. For example, *Salmonella enterica*, which is closely related to human diet, is known as an enteric bacterium inhabiting the intestinal tract of primates including humans, and secretes cytolysin known as an exotoxin. The secreted cytolysin is a cytotoxic protein having a molecular weight of about 34 kDa, and causes hemolysis by destroying red blood cells in the intestines of primates including humans, and forms pores in the membranes of normal cells to induce cell lysis, thereby leading to severe vascular inflammation and local tissue necrosis, which may result in death. However, recent studies have shown that cytolysin isolated and purified from *Salmonella enterica* specifically reacts with cancer tissues present in the intestinal tract in vivo and induces death of cancer tissues, thereby drawing attention as a next-generation anticancer therapeutic agent. Therefore, bacteria transformed with a gene secreting cytolysin, which is a cytotoxic substance, may have very high potential for use as an anticancer therapeutic agent for targeting cancer tissues.

Accordingly, the present inventors have developed a strain having improved anticancer efficacy by additionally introducing, into transformed anaerobic bacteria that can be used as an anticancer therapeutic agent targeting cancer tissues, a gene for expressing a signaling substance for cancer treatment, such as a C-di-AMP synthase gene. Since the DNA construct for expressing a signaling substance for cancer treatment, and the strain transformed with a recombinant vector comprising the DNA construct according to the present invention exhibit remarkable anticancer effects, they are expected to be widely used for cancer treatment in the medical and healthcare fields.

### [Disclosure]

### [Technical Problem]

In one aspect, an object of the present invention is to provide a DNA construct.

In another aspect, an object of the present invention is to provide a recombinant vector comprising the DNA construct.

In still another aspect, an object of the present invention is to provide a strain into which the recombinant vector is introduced.

In still another aspect, an object of the present invention is to provide a pharmaceutical composition for preventing or treating cancer, comprising the strain as an active ingredient.

In still another aspect, an object of the present invention is to provide a method for providing information for diagnosis or treatment of cancer, comprising treating the strain.

However, the technical problems to be achieved by the present invention are not limited to the problems mentioned above, and other problems not mentioned will be clearly understood by those skilled in the art from the following description.

### [Technical Solution]

In one embodiment of the present invention, a DNA construct is provided.

The DNA construct of the present invention comprises a gene encoding a cyclic dinucleotide synthase, and further comprises a secretion signal sequence. The cyclic dinucleotide may be cyclic-di-adenosine monophosphate (C-di-AMP), cyclic-di-diguanylate (C-di-GMP), or cyclic guanosine monophosphate-adenosine monophosphate (cGAMP), and the gene encoding the cyclic dinucleotide synthase may be at least one selected from the group consisting of DisA, CdaA, CdaS, DosC, AdrA, ydeH, YfiN, CGAS, cdnC, relA, capV, cdnD, cdnE, cdnB, AKT1, gdpP, disA, dncV, cdnG, ZDHHC9, dacA, radA, STING3, argF, folP, cdaR, glmS, ybbP, dacA_2, disA_2, ossG, sle_31840, dacB, disA_1, disA_3, 1d0912, dacZ, cGlr1, cGlr2, guaA, cdnA, LYPLAL1, Rnf185, VC_A0931, VC_A0681, GG20550, GD11141, TcasGA2_TC003965, thsB1, eccD1, VC_A0210, CG7194, nadE, STING7, STING2, STING1, STING5, mucR, dgcP, pleD, dgcZ, dgcA, cdgI, dgcM, tpbB, csrA, dgcT, dgcN, dgcQ, dgcS, dgcF, dgcJ, dgcC, dgcE, dgcI, ydiV, vdcA, csrD, dosP, wspR, pdeF, csgD, tpbA, pdeR, and nbdA, but is not limited thereto.

The secretion signal sequence of the present invention facilitates secretion of a protein expressed by a foreign nucleic acid molecule outside bacterial cells, and the foreign nucleic acid molecule may further comprise a leader sequence (or signal sequence). Specifically, it may comprise at least one leader sequence selected from the group consisting of pelB, ompA, ompB, ompC, ompD, ompE, ompF, ompT, phoA, TolB, TorT, LamB, LivK, TorA, SuflCT-B, LTIIb-B, BAP, MF α, SUC2, HasA, PspA, TlyA, OutD, YwbN, NprE, SpsA, LipA, LysK, XcpT, AmyE, EstA, BrkA, IcsA, CelA, ClyA, HlyA, VgrG, EspA, EsxA, AprE, FhuD, MglB, OppA, RbsB, Agp, FkpA, YtfQ, HdeA, HdeB, GlnH, LLO, and phoE, but is not limited thereto.

The DNA construct of the present invention may further comprise at least one selected from the group consisting of a gene encoding an anticancer protein; a gene encoding a cytokine; a gene encoding a chemokine; a gene encoding an immune modulator; an oligonucleotide specific to a cancer antigen; and a gene encoding a reporter protein.

The strain transformed with the DNA construct of the present invention secretes a cyclic dinucleotide synthase to stimulate immunity in a subject to which the strain is administered, and thus may be used as a type of immuno-oncologic agent. In addition, when the strain is an anaerobic bacterium, the strain has a characteristic of preferring an oxygen-deficient environment such as a tumor microenvironment, and thus may be used as a composition for cancer diagnosis. As described above, when the DNA construct according to the present invention is used, cancer diagnosis and treatment may be simultaneously performed.

As used herein, the term "DNA construct" refers to a structure that enables expression of a desired protein, etc., when introduced into a host strain or cell through transformation, and includes not only a gene encoding the desired protein but also a nucleotide sequence corresponding to a promoter, which is an essential regulatory element operably linked so that the gene can be expressed.

As used herein, the term "promoter" refers to a nucleotide sequence located in an upstream region of a gene operably linked in a host strain or cell, and means a nucleotide sequence at a specific site of the DNA construct to which RNA polymerase can bind in order to initiate transcription.

The DNA construct of the present invention may be such that expression of the gene encoding the cyclic dinucleotide synthase is regulated by a cis-acting element or a trans-acting element.

As used herein, the term "regulation" or "expression regulation" may mean activation or suppression of transcription and translation of a specific gene.

The cis-acting element of the present invention is a region of non-coding DNA that regulates transcription of a neighboring gene, is an essential component of a gene regulatory network, and controls gene expression. The cis-acting element may be at least one selected from the group consisting of a ribosome binding site (RBS), a 5'-untranslated region (5'-UTR), a transcription factor binding site, and a terminator, but is not limited thereto.

In the present invention, the ribosome binding site (RBS) is also referred to as a Shine-Dalgarno sequence (SD sequence), and refers to a short sequence present on mRNA that allows a ribosome to effectively bind to the mRNA in order for translation to occur after genetic information contained in DNA is transcribed into messenger RNA (mRNA).

In the present invention, the 5'-untranslated region (5'-UTR) is a region that is not translated and is located on both sides of the coding region, which is a portion translated into amino acids in mRNA at the 5' region, and although it was considered a dispensable portion (junk) during evolution, it is known to play an important role in regulation of gene expression.

In the present invention, the transcription factor binding site is a DNA region that functions to turn on or off a specific nearby gene. The transcription factor binding site may be at least one selected from the group consisting of a promoter, an enhancer, and a silencer of the gene encoding the cyclic dinucleotide synthase, but is not limited thereto.

The promoter for expressing a signaling substance for cancer treatment of the present invention, for example, a promoter of a gene encoding a cyclic dinucleotide synthase, may include any promoter whose activity can be induced under environmental conditions and developmental states of most host strains or cells. For example, it may be at least one selected from the group consisting of an *E. coli* σ70 promoter; an *E. coli* σS promoter; an *E. coli* σ32 promoter; a *B. subtilis* σA promoter; a *B. subtilis* σB promoter; K112706 or K112707, which are promoters derived from *Salmonella*; a bacteriophage T7 promoter; a bacteriophage SP6 promoter; a promoter derived from yeast; I712004 or K076017, which are promoters derived from eukaryotic cells; an OXB1 promoter; and a promoter derived from plants, but is not limited thereto. The *E. coli* σ70 promoter may be at least one selected from the group consisting of I14018, I14033, I14034, I732021, I742126, J01006, J23103, J23109, J23112, J23113, J23117, J23119, J23150, J23151, J44002, J48104, J56015, J64951, K088007, K119000, K119001, K1330002, K137029, K137030, K137031, K137032, K137085, K137086, K137087, K137088, K137089, K137090, K137091, K1585100, K1585101, K1585102, K1585103, K1585104, K1585105, K1585106, K1585110, K1585113, K1585115, K1585116, K1585117, K1585118, K1585119, K2486171, K256002, K256018, K256020, K256033, K292000, K823007, K823010, K823013, M13101, M13102, M13103, M13104, M13105, M13106, M13108, M13110, M31519, R1074, R1075, and S03331, but is not limited thereto. The *E. coli* σS promoter may be J45992 or J45993, but is not limited thereto. The *E. coli* σ32 promoter may be J45504, K1895002, or K1895003, but is not limited thereto. The *B. subtilis* σA promoter may be at least one selected from the group consisting of K143012, K143013, K823000, K823002, and K823003, but is not limited thereto. The *B. subtilis* σB promoter may be K143010, K143011, or K143013, but is not limited thereto. The bacteriophage T7 promoter may be at least one selected from the group consisting of I719005, J34814, J64997, K113010, K113011, K113012, K1614000, R0085, R0180, R0181, R0182, R0183, Z0251, Z0252, and Z0253, but is not limited thereto. The bacteriophage SP6 promoter may be J64998, but is not limited thereto. The promoter derived from yeast may be at least one selected from the group consisting of I766557, J63005, K105027, K105028, K105029, K105030, K105031, K122000, K124000, K124002, K319005, M31201, K2365040, K2365036, K2365041, K2365042, K2365032, K2365051, K2365514, K2365515, and K2365516, but is not limited thereto. The promoter derived from plants may be at least one selected from the group consisting of PLPR0203, PLPR0210, PLPR0177, PLPR0193, PLPR0507, PLPR0422, PLPR0228, PLPR0226, PLPR0223, PLPR0040, PLPR0465, PLPR0232, PLPR0205, PLPR0247, PLPR0328, PLPR0525, AtREG383, AtREG415, AtREG416, OsREG438, OsREG443, OsREG501, PpREG186, PpREG194, and PpREG197, but is not limited thereto.

The enhancer of the present invention is a sequence found in both prokaryotes and eukaryotes, generally having a region of 50 to 1500 bp, and is located upstream or downstream from the start site of a target gene, in the present invention, a signaling substance for cancer treatment, for example, a gene encoding a cyclic dinucleotide synthase, thereby inducing binding of transcription factors.

The silencer of the present invention maintains the same mechanism as the enhancer and acts antagonistically to the enhancer. The transcription factor that binds to the silencer is a repressor. The enhancer and the silencer may be present in adjacent regions to each other, or may be present in the same region but with transcription factors binding to different sites.

The terminator of the present invention is also referred to as a transcription terminator, and mediates termination of transcription of a gene or operon in a genome. In prokaryotes, there are Rho-dependent terminators and Rho-independent terminators.

The trans-acting element of the present invention is also referred to as a transactivating factor or a trans-acting transcription factor, and is a factor that activates transcription of a gene in trans. The trans-acting element may be at least one selected from the group consisting of the transcription factor, an aptamer, sRNA, and antisense RNA (antisense RNA; asRNA), but is not limited thereto.

In the present invention, the transcription factor is a protein that binds to the transcription factor binding site and helps turn on or off a specific gene.

In the present invention, the aptamer is a part of a riboswitch and collectively refers to an oligonucleotide or peptide capable of binding to a specific target molecule, and the aptamer may be a peptide aptamer or a nucleic acid aptamer. The riboswitch is a type of mRNA that regulates gene expression, and may include, but is not limited to, a glmS riboswitch, an FMN riboswitch, and a cobalamin riboswitch.

In the present invention, the sRNA and antisense RNA (asRNA) refer to single-stranded RNA capable of binding complementarily to a specific RNA. By binding complementarily to sense RNA, which is messenger RNA (mRNA) expressing a specific protein, the expression of the corresponding protein is ultimately regulated.

As used herein, the term "operably linked" means that one nucleic acid fragment of interest is functionally linked to another nucleic acid fragment such that the function or expression of the one nucleic acid fragment of interest is affected by the other nucleic acid fragment.

As used herein, the term "reporter protein" refers to a protein that performs a function of enabling visual diagnosis of cancer, and may be at least one selected from the group consisting of a fluorescent protein, luciferase, and proteins used for nuclear medicine or MRI imaging, but is not limited thereto.

As used herein, the term "fluorescent protein" refers to a protein that inherently emits fluorescence so that cancer can be visually diagnosed, and may be at least one selected from the group consisting of green fluorescent protein (GFP), modified green fluorescent protein (MGFP), enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), and enhanced yellow fluorescent protein (EYFP), but is not limited thereto.

The proteins used for nuclear medicine or MRI imaging of the present invention may be at least one selected from the group consisting of herpes simplex virus thymidine kinase , a dopamine receptor, a somatostatin receptor, a sodium-iodide transporter, an iron receptor, a transferrin receptor, ferritin, and an iron transporter (magA), but is not limited thereto.

As used herein, the term "cytokine" refers to a protein secreted by immune cells, and the cytokine of the present invention may include any cytokine that can be used in cancer immunotherapy capable of inducing death of disease-related cells, for example, cancer cells, by regulating a host immune response, and preferably may be IFN-α2, IL-2, IL-15, IL-21, and IL-12, but is not limited thereto.

As used herein, the term "chemokine" refers to a factor that regulates cell migration between tissues and the positioning and interaction of cells within tissues, and may include any chemokine capable of mediating a host response to a disease, for example, cancer, by inducing leukocytes into a tumor microenvironment, and preferably may be CXCR3, CCR5, and the like, but is not limited thereto.

As used herein, the term "immune modulator" refers to a factor that enables various treatments by utilizing an inherent immune system of a subject, and may include any immune modulator capable of activating immune cells to induce death of disease-related cells, for example, cancer cells.

As used herein, the term "anticancer protein" refers to a peptide having a function capable of directly or indirectly inducing death of cancer cells, and may be at least one selected from the group consisting of a toxin protein; an antibody specific to a cancer antigen or a fragment of the antibody; a tumor suppressor protein; an angiogenesis inhibitor; a cancer antigen; a prodrug-converting enzyme; a tumor microenvironment matrix-degrading enzyme; and a pro-apoptotic protein, but is not limited thereto.

As used herein, the term "toxin protein" refers to a protein having a function capable of directly or indirectly inducing death of cancer cells, and may be at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA), and more preferably may be cytolysin A, but is not limited thereto.

As used herein, the term "tumor suppressor protein" refers to a protein encoded by a gene that normally exists in normal cells and maintains its function, but when the function is lost, induces uncontrolled cell division and growth of normal cells, thereby causing transformation into cancer cells, and may be, for example, Retinoblastoma protein (RB), p53 protein, Adenomatous polyposis coli (APC) protein, Phosphatase and tensin homologue (PTEN) protein, and cyclin dependent kinase inhibitor 2A (CDKN2A) protein, but is not limited thereto.

The antibody specific to a cancer antigen or a fragment thereof of the present invention refers to an antibody capable of specifically binding to an antigen that is highly expressed on the surface or in the cytoplasm of cancer cells, and may be, for example, an antibody specific to HER2, which is highly expressed in breast cancer or gastric cancer cells, but is not limited thereto.

The antibody of the present invention refers to a protein molecule capable of specifically binding to an antigenic site of a protein or peptide molecule. The form of the antibody is not particularly limited, and any type of immunoglobulin antibody may be included, as long as it has antigen-binding activity, including polyclonal antibodies, monoclonal antibodies, or even a portion of an antibody. In addition, special antibodies such as humanized antibodies may be included, and the antibody includes not only a complete form having two full-length light chains and two full-length heavy chains, but also functional fragments of an antibody molecule. The functional fragment of an antibody molecule refers to a fragment retaining at least antigen-binding function, and may include Fab, F(ab'), F(ab')₂, Fv, and the like, but is not limited thereto.

As used herein, the term "antibody" may be prepared by obtaining a protein encoded by a gene encoding the cancer antigen of the present invention by cloning the gene into an expression vector according to a conventional method, followed by preparation using a conventional method.

As used herein, the term "angiogenesis inhibitor" refers to a protein or compound having a function capable of directly or indirectly inducing death of cancer cells by inhibiting the formation of new blood vessels around cancer cells. Preferably, the angiogenesis inhibitor may be angiostatin, endostatin, thrombospondin, and a protease inhibitor protein, but is not limited thereto.

As used herein, the term "cancer antigen" refers to a protein capable of directly or indirectly inducing death of cancer cells by inducing an anti-tumor immune response using, as an antigen, a protein that is expressed in cancer cells but is scarcely expressed in normal cells. Preferably, the cancer antigen of the present invention may be α-fetoprotein (AFP), vascular endothelial growth factor receptor 2 (VEGFR2), survivin, legumain, prostate cancer specific antigen (PCSA), and the like, but is not limited thereto.

As used herein, the term "prodrug-converting enzyme" refers to a protein having a function of converting an inactive drug into an active drug through metabolism by an enzymatic reaction. When such a prodrug-converting enzyme is used, the inactive drug is metabolized and converted into an active drug capable of directly or indirectly inducing death of cancer cells, and thus may be very usefully used for prevention or treatment of cancer. Preferably, the prodrug-converting enzyme of the present invention may be thymidine kinase, cytosine deaminase, nitroreductase, purine nucleoside phosphorylase, carboxypeptidase G2, chromate reductase YieF, herpes simplex virus type I thymidine kinase/ganciclovir (HSV1-TK/GCV), β-glucuronidase, and the like, but is not limited thereto.

As used herein, the term "tumor microenvironment matrix-degrading enzyme" refers to an enzyme capable of degrading a matrix present in a tumor microenvironment, that is, a cellular environment in which a tumor exists, including surrounding blood vessels, immune cells, fibroblasts, and other cells that send signals to molecules and the extracellular matrix. Preferably, the tumor microenvironment matrix-degrading enzyme of the present invention may be collagenase, heparinase, hyaluronidase, protease, chymotrypsin, chymopapain, trypsin, caseinase, elastase, papain, deoxyribonuclease, matrix metalloproteinases (MMPs), cathepsins, β-glucuronidase, serine proteases, and the like, but is not limited thereto.

As used herein, the term "pro-apoptotic protein" refers to a protein that induces direct or indirect death of cancer cells by causing deficiency of factors (proteins, nutrients, oligonucleotides, etc.) essential for growth or maintenance of cancer cells. Preferably, the pro-apoptotic protein of the present invention may be L-asparaginase (L-ASNase), RNA-binding motif protein 5 (RBM5), and the like, but is not limited thereto.

The oligonucleotide specific to a cancer antigen of the present invention refers to a nucleotide capable of inhibiting expression or function of the cancer antigen by binding complementarily to a gene or mRNA of the cancer antigen, and may be any one selected from the group consisting of an antisense oligonucleotide, an aptamer, siRNA, and shRNA, but is not limited thereto.

As used herein, the term "antisense oligonucleotide" refers to DNA, RNA, or a derivative thereof comprising a nucleic acid sequence complementary to a sequence of a specific mRNA, and may inhibit translation of the mRNA into a protein by binding to a complementary sequence within the mRNA. The antisense oligonucleotide may be synthesized in vitro using a conventional method, for example, using RNA polymerase I, followed by administration in vivo, or may be synthesized in vivo by using a method such as employing a vector in which a multiple cloning site (MCS) is oriented in the opposite direction.

As used herein, the term "aptamer" refers to a small single-stranded oligonucleic acid capable of specifically recognizing a target substance with high affinity. For the purposes of the present invention, the target substance may be a gene or mRNA of a cancer antigen.

As used herein, the term "siRNA" refers to a short double-stranded RNA capable of inducing an RNA interference (RNAi) phenomenon through cleavage of a specific mRNA. It consists of a sense RNA strand having a sequence homologous to mRNA of a target gene and an antisense RNA strand having a sequence complementary thereto, and for the purposes of the present invention, the siRNA may specifically bind to mRNA transcribed from a gene encoding a cancer antigen to effectively inhibit expression of such a gene.

As used herein, the term "shRNA" refers to short hairpin RNA, which has advantages over siRNA in that it has a higher transfection efficiency and allows RNA interference to be maintained for a longer period of time. RNA interference may be induced through a process of transforming adenovirus, lentivirus, and plasmid expression vector systems into cells from a promoter of RNA polymerase III and expressing the same, but is not limited thereto. For the purposes of the present invention, the shRNA may specifically bind to mRNA transcribed from a gene encoding a cancer antigen to effectively inhibit expression of such a gene.

In another embodiment of the present invention, a recombinant vector comprising the DNA construct of the present invention is provided.

The recombinant vector of the present invention comprises the DNA construct of the present invention, such that a signaling substance for cancer treatment inherent in the DNA construct, for example, a gene encoding a cyclic dinucleotide synthase, is expressed by a separate promoter, and may be introduced into a host cell or strain to transform the host cell or strain.

In the recombinant vector of the present invention, descriptions of the DNA construct, anticancer protein, cytokine, chemokine, immune modulator, oligonucleotide specific to a cancer antigen, reporter protein, and promoter are the same as those described for the DNA construct, and thus are omitted herein to avoid excessive complexity of the present specification.

The recombinant vector of the present invention is a means for expressing a protein by being introduced into a cell, and known recombinant vectors such as plasmid vectors, cosmid vectors, and bacteriophage vectors may be used, and the recombinant vector may be easily prepared by those skilled in the art according to any known method using DNA recombination techniques.

Specific examples of the recombinant vector of the present invention may include, but are not limited to, commercially widely used vectors such as pCDNA vectors; F, R1, RP1, Col, pBR322, ToL, Ti vectors; cosmids; phages such as lambda, lambdoid, M13, Mu, P1, P22, Qβ, T-even, T2, T3, and T7; and plant viruses. For the purposes of the present invention, an appropriate recombinant vector may be selected depending on the properties of the host cell or strain.

In still another embodiment of the present invention, a host cell or a strain into which a recombinant vector comprising the DNA construct of the present invention is introduced is provided.

The host cell of the present invention may include cells of mammalian, plant, insect, fungal, or cellular origin, and examples thereof may include bacterial cells such as *E. coli*, *Streptomyces*, or *Salmonella* genus strains; fungal cells such as yeast cells and *Pichia pastoris*; insect cells such as *Drosophila* and *Spodoptera* Sf9 cells; animal cells such as CHO (Chinese hamster ovary cells), SP2/0 (mouse myeloma), human lymphoblastoid cells, COS cells, NSO (mouse myeloma), 293T cells, B16 melanoma cells, HT-1080 cells, BHK cells (Baby Hamster Kidney cells), HEK cells (Human Embryonic Kidney cells), and PER.C6 cells (human retinal cells); and plant cells, and may be at least one selected from the group consisting thereof, but is not limited thereto. For the purposes of the present invention, the strain may be an anaerobic strain, for example, at least one selected from the group consisting of *Salmonella* genus strains, *Clostridium* genus strains, *Bifidobacterium* genus strains, and *E. coli* strains, and preferably may be at least one selected from the group consisting of *Salmonella typhimurium*, *Salmonella choleraesuis*, and *Salmonella enteritidis*, and more preferably may be *Salmonella typhimurium*, but is not limited thereto.

The strain of the present invention may be attenuated.

As used herein, the term "attenuated" means that a microorganism has been genetically modified, for example, by modifying genes, so as to reduce toxicity and other side effects when administered to a patient. For the purposes of the present invention, when the strain is a *Salmonella* genus strain, the attenuation may be achieved by modifying at least one gene selected from the group consisting of aroA, aroC, aroD, aroE, Rpur, htrA, ompR, ompF, ompC, galE, cya, crp, cyp, phoP, phoQ, rfaY, dksA, hupA, sipC, clpB, clpP, clpX, pab, nadA, pncB, pmi, rpsL, hemA, rfc, poxA, galU, cdt, pur, ssa, guaA, guaB, fliD, flgK, flgL, relA, and spoA, but is not limited thereto.

The method of modifying the gene of the present invention may be performed by various methods known in the art for deletion or disruption of genes, for example, the deletion or disruption may be performed by methods such as homologous recombination, chemical mutagenesis, radiation mutagenesis, or transposon mutagenesis.

In the present invention, since the strain targets the interior of cancer tissues, which are environments with incomplete angiogenesis and oxygen deficiency that are highly suitable for proliferation of anaerobic strains, when a recombinant vector capable of simultaneously and balanced expression of a reporter protein enabling real-time imaging and an anticancer protein is introduced into such a strain, cancer can be very effectively diagnosed and treated simultaneously.

In the strain of the present invention, descriptions of the DNA construct, anticancer protein, cytokine, chemokine, immune modulator, oligonucleotide specific to a cancer antigen, reporter protein, promoter, and recombinant vector are the same as those described for the DNA construct and the recombinant vector, and thus are omitted herein to avoid excessive complexity of the present specification.

The recombinant vector of the present invention may be introduced into a host cell or strain through transformation (or transfection), and any transformation method may be used in the present invention, and may be easily performed according to methods commonly used in the art. Specifically, the recombinant vector may be introduced into the strain using commonly used transformation methods for bacteria such as *Salmonella* genus strains, a CaCl₂ precipitation method, a Hanahan method in which efficiency is increased by using a reducing agent, dimethyl sulfoxide (DMSO), in the CaCl₂ method, electroporation, a calcium phosphate precipitation method, a protoplast fusion method, an agitation method using silicon carbide fibers, an *Agrobacterium*-mediated transformation method, a polyethylene glycol (PEG)-mediated transformation method, dextran sulfate, lipofectamine, and a drying/inhibition-mediated transformation method, but is not limited thereto.

In still another embodiment of the present invention, a pharmaceutical composition for preventing or treating cancer is provided.

The pharmaceutical composition of the present invention comprises, as an active ingredient, the DNA construct of the present invention, or a strain transformed with a vector comprising the DNA construct.

The strain of the present invention is transformed with the DNA construct according to the present invention, targets cancer in a subject, and then secretes a signaling substance for cancer treatment, for example, a cyclic dinucleotide synthase, in the environment surrounding the cancer, and thus can very effectively prevent or treat cancer and, at the same time, diagnose cancer in real time.

As used herein, the term "cancer" refers to a disease characterized by rapid and uncontrolled growth of abnormal cells, and may be at least one selected from the group consisting of melanoma, fallopian tube cancer, brain cancer, small intestine cancer, esophageal cancer, lymphoma, gallbladder cancer, blood cancer, thyroid cancer, endocrine cancer, oral cancer, liver cancer, bile duct cancer, colorectal cancer, rectal cancer, cervical cancer, ovarian cancer, kidney cancer, gastric cancer, duodenal cancer, prostate cancer, breast cancer, brain tumor, lung cancer, anaplastic thyroid cancer, uterine cancer, colon cancer, bladder cancer, ureter cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma, and preferably may be at least one selected from the group consisting of liver cancer, bile duct cancer, colorectal cancer, rectal cancer, cervical cancer, ovarian cancer, kidney cancer, gastric cancer, duodenal cancer, prostate cancer, breast cancer, brain tumor, lung cancer, anaplastic thyroid cancer, uterine cancer, colon cancer, bladder cancer, ureter cancer, pancreatic cancer, bone/soft tissue sarcoma, and skin cancer, and more preferably may be colon cancer, but is not limited thereto.

As used herein, the term "prevention" refers to any action that blocks, suppresses, or delays symptoms caused by cancer using the active ingredient of the present invention, without limitation.

As used herein, the term "treatment" refers to any action in which symptoms caused by cancer are ameliorated or beneficial effects occur in a subject by using the active ingredient of the present invention, and includes attempts to obtain useful or desired results, including clinical results. Useful or desired clinical results may include, but are not limited to, alleviation or improvement of one or more symptoms or conditions, whether detectable or not, reduction in the extent of disease, stabilization of a disease state, inhibition of disease occurrence, inhibition of disease spread, delay or slowing of disease progression, delay or slowing of disease onset, improvement or alleviation of a disease state, and remission (partial or complete). In addition, "treatment" may mean prolongation of survival of a patient beyond what would be expected in the absence of treatment. Furthermore, "treatment" may mean inhibition of disease progression or temporary delay of disease progression, and more preferably may be associated with permanently arresting the progression of the disease. As understood by those skilled in the art, if treatment results in an adverse outcome in a treated patient that outweighs all benefits conferred by the treatment while improving a particular disease condition, the outcome may not be beneficial or desirable.

In the pharmaceutical composition of the present invention, descriptions of the DNA construct, anticancer protein, cytokine, chemokine, immune modulator, oligonucleotide specific to a cancer antigen, reporter protein, promoter, recombinant vector, strain, and transformation are the same as those described for the DNA construct, recombinant vector, and strain, and thus are omitted herein to avoid excessive complexity of the present specification.

The pharmaceutical composition of the present invention may be in the form of a capsule, tablet, granule, injectable preparation, ointment, powder, or beverage, and may be intended for use in humans.

The pharmaceutical composition of the present invention is not limited thereto, but may be formulated and used in the form of oral formulations such as powders, granules, capsules, tablets, and aqueous suspensions, as well as external preparations, suppositories, and sterile injectable solutions, according to conventional methods. The pharmaceutical composition of the present invention may comprise a pharmaceutically acceptable carrier. Pharmaceutically acceptable carriers may include, for oral administration, binders, lubricants, disintegrants, excipients, solubilizers, dispersants, stabilizers, suspending agents, colorants, and flavoring agents, and for injectable preparations, buffers, preservatives, analgesics, solubilizers, isotonic agents, and stabilizers may be mixed and used, and for topical administration, bases, excipients, lubricants, and preservatives may be used. The formulations of the pharmaceutical composition of the present invention may be prepared in various forms by mixing with pharmaceutically acceptable carriers as described above. For example, for oral administration, the composition may be prepared in the form of tablets, troches, capsules, elixirs, suspensions, syrups, wafers, and the like, and for injectable preparations, it may be prepared in the form of unit dosage ampules or multiple dosage forms. In addition, it may be formulated into solutions, suspensions, tablets, capsules, sustained-release formulations, and the like.

Meanwhile, examples of carriers, excipients, and diluents suitable for formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil. In addition, fillers, anti-caking agents, lubricants, wetting agents, flavoring agents, emulsifiers, and preservatives may be further included.

The route of administration of the pharmaceutical composition of the present invention is not particularly limited, but may include oral, intravenous, intramuscular, intra-arterial, intraosseous, intradermal, intracardiac, transdermal, subcutaneous, intraperitoneal, intranasal, enteral, topical, sublingual, or rectal administration. Oral or parenteral administration is preferred.

As used herein, the term "parenteral" includes subcutaneous, intradermal, intravenous, intramuscular, intra-articular, intrabursal, intrasternal, intrathecal, intralesional, and intracranial injection or infusion techniques. The pharmaceutical composition of the present invention may also be administered in the form of a suppository for rectal administration.

The dosage of the pharmaceutical composition of the present invention may vary depending on various factors including the activity of the specific compound used, age, body weight, general health, sex, diet, time of administration, route of administration, excretion rate, drug combination, and severity of the specific disease to be prevented or treated, and although the dosage may vary depending on the condition of the patient, body weight, severity of disease, dosage form, route and period of administration, it may be appropriately selected by those skilled in the art, and may be administered in an amount of 0.0001 to 50 mg/kg or 0.001 to 50 mg/kg per day. The administration may be performed once a day or may be divided into several times per day. The dosage does not limit the scope of the present invention in any way. The pharmaceutical composition according to the present invention may be formulated into pills, sugar-coated tablets, capsules, liquid preparations, gels, syrups, slurries, or suspensions.

In still another embodiment of the present invention, a composition for diagnosing cancer is provided.

The diagnostic composition of the present invention comprises, as an active ingredient, the DNA construct according to the present invention, or a strain transformed with a vector comprising the DNA construct, wherein the strain may further comprise a reporter protein enabling tracking of the strain.

The strain of the present invention is transformed with the DNA construct according to the present invention, targets cancer in a subject, and then secretes a signaling substance for cancer treatment, for example, a cyclic dinucleotide synthase, in the environment surrounding the cancer, and thus can very effectively prevent or treat cancer and, at the same time, diagnose cancer in real time.

As used herein, the term "diagnosis" refers to any act of identifying cancer tissues in vivo, including monitoring the presence or absence of cancer in real time by a reporter protein expressed from the DNA construct introduced into the strain when the strain targets and localizes to cancer.

In the diagnostic composition of the present invention, descriptions of the DNA construct, anticancer protein, reporter protein, constitutive promoter, inducible promoter, recombinant vector, *Salmonella* genus strain, transformation, cancer, and the like are the same as those described for the DNA construct, recombinant vector, strain, and pharmaceutical composition, and thus are omitted herein to avoid excessive complexity of the present specification.

In still another embodiment of the present invention, a method for providing information for diagnosis or treatment of cancer is provided.

The method of the present invention comprises treating a biological sample isolated from a subject with a strain into which the recombinant vector according to the present invention is introduced.

The method for providing information for diagnosis of cancer of the present invention may further comprise diagnosing cancer when a reporter protein is expressed from the strain.

As used herein, the term "biological sample" refers to any material, tissue, or cell obtained from or derived from a subject, and may include, for example, a tissue, a cell, or a cell extract, but is not limited thereto.

In the method for providing information for diagnosis of the present invention, descriptions of the DNA construct, anticancer protein, cytokine, chemokine, immune modulator, oligonucleotide specific to a cancer antigen, reporter protein, promoter, recombinant vector, strain, transformation, cancer, diagnosis, and the like are the same as those described for the DNA construct, recombinant vector, strain, pharmaceutical composition, and diagnostic composition, and thus are omitted herein to avoid excessive complexity of the present specification.

### [Advantageous Effects]

The DNA construct according to the present invention, or a strain transformed with a vector comprising the DNA construct, targets cancer in a subject and then secretes a cyclic dinucleotide synthase in the environment surrounding the cancer, and thus can very effectively prevent or treat cancer and, at the same time, diagnose cancer in real time.

In addition, since the DNA construct of the present invention does not allow expression of the cyclic dinucleotide synthase at all in the absence of doxycycline, cyclic dinucleotides can be synthesized at an appropriate dose for cancer treatment by controlling administration of doxycycline, and at the same time, the size of cancer can be monitored in real time according to the expression level of a reporter protein.

### [Description of Drawings]

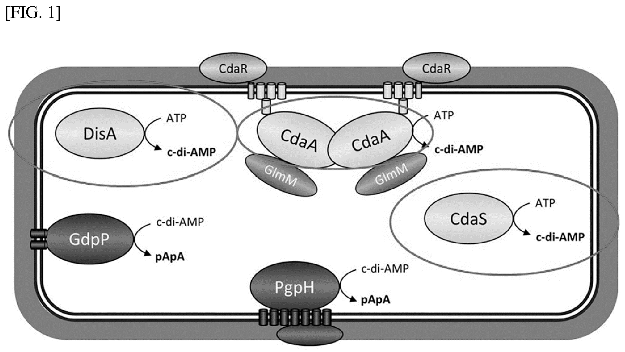

Fig. 1 is a schematic diagram of a mechanism by which DisA, CdaA, and CdaS genes generate a cyclic dinucleotide (C-di-AMP) in bacteria according to an embodiment of the present invention.
Fig. 2 shows results confirming that a cyclic dinucleotide is produced in a strain transformed with a DNA construct comprising a gene encoding a cyclic dinucleotide synthase according to an embodiment of the present invention.
Fig. 3 shows results confirming that transcription of IFNb and CXCL10 is increased by a cyclic dinucleotide according to an embodiment of the present invention.
Fig. 4 is a schematic diagram of an animal experiment for confirming the anticancer effect of a strain transformed with a DNA construct comprising a gene encoding a cyclic dinucleotide synthase according to an embodiment of the present invention.
Fig. 5 shows results confirming a tumor suppression effect of a strain transformed with a DNA construct comprising a gene encoding a cyclic dinucleotide synthase according to an embodiment of the present invention.
Fig. 6 shows results confirming an effect of improving survival rate of tumor-bearing subjects by a strain transformed with a DNA construct comprising a gene encoding a cyclic dinucleotide synthase according to an embodiment of the present invention.
Fig. 7 shows results confirming a rapid decrease in the strain and a rapid increase in expression of IFN-b in tumors in some mice administered with a strain transformed with a DNA construct comprising a gene encoding a cyclic dinucleotide synthase according to an embodiment of the present invention.
Fig. 8 shows results confirming changes in the amount of a strain when a strain transformed with a DNA construct comprising a gene encoding a cyclic dinucleotide synthase is cultured in an animal cell culture medium according to an embodiment of the present invention.
Fig. 9 shows results confirming changes in the amount of a strain when a strain transformed with a DNA construct comprising a gene encoding a cyclic dinucleotide (C-di-GMP) synthase is cultured in an animal cell culture medium according to an embodiment of the present invention.
Fig. 10 shows results confirming changes in the amount of a strain when a strain transformed with a DNA construct comprising a gene encoding a cyclic dinucleotide (cGAMP) synthase is cultured in an animal cell culture medium according to an embodiment of the present invention.
Fig. 11 shows results confirming changes in the amount of a strain when a strain transformed with a DNA construct comprising a cyclic dinucleotide gene is cultured in media having various salt concentrations according to an embodiment of the present invention.
Fig. 12 shows results confirming antibiotic sensitivity by measuring a size of a clear zone formed by various antibiotic concentrations when a strain transformed with a DNA construct comprising a gene encoding a cyclic dinucleotide synthase is cultured in a medium according to an embodiment of the present invention.
Fig. 13 shows results confirming the potential of the strain of the present invention as a system for releasing and delivering foreign proteins or nucleic acid molecules that cannot be secreted from bacteria, wherein it was confirmed that GFP was not secreted when expressed alone, but was released into the medium upon expression of DisA according to an embodiment of the present invention.
Fig. 14 shows results confirming expression of C-di-AMP in a strain transformed with a DNA construct comprising a secretion gene and a gene encoding a cyclic dinucleotide synthase according to an embodiment of the present invention.
Fig. 15 shows results confirming whether endogenous proteins are secreted in a strain transformed with a DNA construct comprising a secretion gene and a gene encoding a cyclic dinucleotide synthase according to an embodiment of the present invention.
Fig. 16 shows results confirming changes in the amount of a strain when a strain transformed with a DNA construct comprising a secretion gene and a gene encoding a cyclic dinucleotide synthase is cultured in an animal cell culture medium according to an embodiment of the present invention.
Fig. 17 shows results confirming antibiotic sensitivity by culturing a strain transformed with a DNA construct comprising a secretion gene and a gene encoding a cyclic dinucleotide synthase in media treated with antibiotics at various concentrations according to an embodiment of the present invention.
Fig. 18 shows results confirming evaluation of antibiotic sensitivity of a strain transformed with a DNA construct comprising a secretion gene and a gene encoding a cyclic dinucleotide synthase according to an embodiment of the present invention.
Fig. 19 shows a tumor energy depletion mechanism of a strain transformed with a DNA construct comprising a secretion gene and a gene encoding a cyclic dinucleotide synthase according to an embodiment of the present invention.
Fig. 20 shows results confirming an immune-stimulating effect of a strain transformed with a DNA construct comprising a secretion gene and a gene encoding a cyclic dinucleotide synthase by IFN-β according to an embodiment of the present invention.
Fig. 21 shows results confirming an immune-stimulating effect of a strain transformed with a DNA construct comprising a secretion gene and a gene encoding a cyclic dinucleotide synthase by TNFα according to an embodiment of the present invention.
Fig. 22 shows results of measuring ATP production in a medium in which a strain transformed with a DNA construct comprising a secretion gene and a gene encoding a cyclic dinucleotide synthase is cultured according to an embodiment of the present invention.
Fig. 23 shows results of measuring the degree of ATP depletion after treatment with a strain transformed with a DNA construct comprising a secretion gene and a gene encoding a cyclic dinucleotide synthase according to an embodiment of the present invention.
Fig. 24 shows results confirming the amount of adenosine production after treatment with a strain transformed with a DNA construct comprising a secretion gene and a gene encoding a cyclic dinucleotide synthase according to an embodiment of the present invention.
Fig. 25 is a schematic diagram of an animal experiment for confirming the anticancer effect of a strain transformed with a DNA construct comprising a secretion gene and a gene encoding a cyclic dinucleotide synthase according to an embodiment of the present invention.
Fig. 26 shows results confirming a tumor suppression effect of a strain transformed with a DNA construct comprising a secretion gene and a gene encoding a cyclic dinucleotide synthase according to an embodiment of the present invention.
Fig. 27 shows results confirming P-gp expression and intracellular accumulation of doxorubicin in a strain transformed with a DNA construct comprising a secretion gene and a gene encoding a cyclic dinucleotide synthase according to an embodiment of the present invention.
Fig. 28 shows results confirming tumor cell viability upon treatment with a strain transformed with a DNA construct comprising a secretion gene and a gene encoding a cyclic dinucleotide synthase according to an embodiment of the present invention.
Fig. 29 shows results confirming viability of doxorubicin-resistant tumor cells upon treatment with a strain transformed with a DNA construct comprising a secretion gene and a gene encoding a cyclic dinucleotide synthase according to an embodiment of the present invention.
Fig. 30 shows results confirming viability of cisplatin-resistant tumor cells upon treatment with a strain transformed with a DNA construct comprising a secretion gene and a gene encoding a cyclic dinucleotide synthase.

### [Best Mode]

In order to confirm whether *Salmonella* strains transformed with the improved DNA construct expressing a STING agonist synthase induce anticancer effects in living animal subjects, tumor animal model experiments were performed while administering doxycycline. The overall in vivo experimental setup was the same as that of Example 2-3. As a result, it was confirmed that the pJH18_pDisA strain exhibited very excellent tumor suppression effects.

### [Mode for Invention]

Hereinafter, the present invention will be described in more detail through examples. These examples are provided only for the purpose of describing the present invention more specifically, and it will be apparent to those skilled in the art that the scope of the present invention is not limited by these examples in accordance with the gist of the present invention.

### Examples

### Example 1. Construction of a DNA construct expressing a STING agonist synthase

STING (stimulator of interferon genes), a protein present in immune cells, functions as a sensor that detects cancer cells and prepares immune cells to attack the cancer cells. In addition, cyclic dinucleotides such as c-di-AMP, c-di-GMP, and cGAMP act as STING agonists to activate the STING pathway and induce interferon production, thereby enhancing anticancer immune responses. Accordingly, the present inventors sought to develop recombinant strains transformed to express enzymes synthesizing C-di-AMP, c-di-GMP, and cGAMP, and to use the same for cancer treatment.

To this end, DisA, CdaA, CdaS, DosC, AdrA, ydeH, YfiN, Adra, DosC(S), CGAS, or Cgas were selected as genes encoding cyclic dinucleotide (C-di-AMP, C-di-GMP, or cGAMP) synthases, and their gene sequences are shown in Table 1. These were amplified using forward primers (F) and reverse primers (R) described in Table 2, and the amplification products were digested with restriction enzymes and purified to obtain gene amplification products, followed by introduction thereof into a pJH18 plasmid to construct a plasmid.

**[Table 1]**

| **Target gene (Sequence No.)** |
|---|
| Sequence |
| **DisA(Sequence No. 1)** |
| ATGGAAAAAGAGAAAAAAGGGGCGAAACACGAGTTAGACCTGTCATCTATAT TGCAGTTTGTTGCTCCGGGTACACCGCTCAGAGCGGGGATGGAAAACGTCTT GAGAGCAAATACAGGCGGTCTGATTGTTGTTGGATATAATGATAAAGTAAAAG AAGTGGTGGACGGCGGCTTTCACATAAACACGGCTTTTTCTCCGGCGCATTTA TATGAGCTGGCTAAAATGGATGGAGCGATCATTTTAAGTGATTCTGGTCAAAA GATCCTATACGCGAATACTCAGCTGATGCCGGATGCCACAATTTCTTCATCAGA AACAGGAATGCGGCACAGAACTGCCGAAAGAGTAGCTAAGCAAACTGGCTG TCTTGTAATCGCCATTTCTGAAAGAAGAAATGTCATAACGTTATATCAGGAAA ACATGAAGTATACACTAAAAGACATAGGATTTATTTTAACCAAGGCGAACCAA GCCATTCAAACACTTGAAAAATATAAGACAATCCTCGATAAAACGATTAATGC ACTGAACGCGTTAGAGTTTGAGGAACTTGTTACCTTCAGTGATGTCTTGTCTG TCATGCATCGTTATGAAATGGTACTGAGAATCAAAAACGAAATTAATATGTATA TCAAAGAGCTGGGGACAGAAGGGCATCTGATCAAACTGCAAGTCATTGAATT GATTACGGATATGGAAGAAGAGGCCGCTTTATTTATTAAGGACTATGTAAAAG AAAAGATTAAAGATCCGTTTGTTCTCTTGAAGGAGCTGCAGGATATGTCCAGT TATGATCTGCTGGATGATTCCATTGTGTATAAGCTTCTCGGTTACCCTGCTTCTA CTAATCTTGATGATTATGTATTGCCGAGAGGATACAGGCTGTTAAATAAGATAC CGCGTCTTCCGATGCCGATTGTTGAAAATGTTGTAGAAGCATTTGGAGTCCTG CCAAGGATTATTGAGGCGAGTGCAGAAGAATTAGATGAAGTAGAGGGAATCG GTGAAGTACGAGCCCAAAAAATCAAAAAAGGATTAAAACGCCTGCAAGAGA AGCATTATTTAGACAGACAACTGTGA |
| **CdaA(Sequence No. 2)** |
| ATGGCTTTTGAGGATATCCCTTTTTTGCAGTACCTCGGCAATGCCGTTGATAT TCTCCTTGTTTGGTATGTGATATATAAATTGATTATGGTGATACGCGGCACGA AAGCGGTTCAGCTGTTAAAAGGAATTGTAGTCATCGTGCTTGTTCGTATGGC CAGCCAATATTTGGGCCTCAGCACACTTCAATGGCTGATGGACCAAGCGATA ACATGGGGATTTTTAGCAATTATTATTATTTTTCAGCCTGAGCTGAGAAGAG CGCTTGAACAGCTAGGCCGCGGCCGCTTTTTTTCGAGGAGCGGCACGCCTGT TGAAGAAGCGCAGCAGAAAACGATTGAGGCCATTACAAAAGCAATCAATTA TATGGCGAAACGCCGTATAGGCGCCCTGCTGACCATTGAGCGGGATACTGG AATGGGCGATTATATTGAGACCGGCATACCACTAAATGCCAAAGTCAGCTCT GAGCTGCTGATCAATATTTTTATTCCAAACACCCCGCTTCATGACGGTGCGG TAATTATGAAAAATAATGAAATTGCCGCGGCCGCCTGTTATCTGCCGCTTTC TGAGAGCCCGTTTATTTCAAAAGAACTGGGCACGCGGCACAGAGCAGCTGT CGGTATCAGTGAAGTAACAGACAGTTTGACGATTATTGTGTCTGAAGAGACC GGCGGCGTCAGTGTGGCAAAGAACGGCGACCTTCACAGAGAGCTTACAGAA GAAGCGCTGAAAGAAATGCTTGAAGCCGAGTTTAAGAAAAACACCAGAGAC ACTTCTTCTAACCGCTGGTATTGGAGGGGCAGGAAAAATGGATAA |
| **CdaS(Sequence No. 3)** |
| ATGAAGGCTATGCGCTATGAGCAGATATCAGAAAATGCGTTCAAGGGGAAA ATACAGGTGTACTTAGAGCAAATATTAGGTGATGCATCTCTAATCTTGAAAA CGCTTCACGAAAAAGACCAGTGTCTCCTTTGTGAATTAGATGATTTGGGGCA CGTTTTTCAGGACATGCAGGGGATTGCATCCTCCTTTTATTTGCAATCATACA TTGAGGAATTTACGCCGGCATTTATTGAGCTGGCCAAGGCGATTAAAGCCTT ATCGGAACATAAGCATGGAGCTTTAATTGTGATTGAAAGAGCAGACCCGGT TGAACGTTTTATTCAAAAAGGCACCTCCCTACACGCCGAGATCAGCTCTTCA TTAATTGAAAGCATCTTTTTTCCCGGAAACCCTCTTCATGACGGCGCTTTATT AGTGAGGGAAAACAAACTCGTGTCAGCAGCCAATGTGCTCCCGCTGACGAC GAAGGAAGTGGATATCCATTTGGGAACCCGGCACCGGGCGGCTCTTGGTAT GTCGGGCTATACGGATGCACTGGTGCTCGTGGTTTCAGAGGAAACAGGGAA GATGTCGTTTGCCAAAGACGGTGTATTATATCCGTTAATTTCACCAAGAACG TAA |
| **DosC(Sequence No. 4)** |
| ATGGAGATGTATTTTAAAAGAATGAAAGATGAGTGGACCGGACTTGTCGAA CAGGCAGATCCGCCCATTCGTGCTAAAGCCGCGGAAATTGCCGTTGCGCATG CTCATTATCTGAGTATCGAGTTTTATCGAATTGTCCGCATCGACCCGCATGCC GAAGAATTCTTGAGTAATGAACAAGTTGAGCGGCAGTTGAAGAGTGCGATG GAACGCTGGATTATTAACGTGCTTTCTGCCCAGGTTGACGATGTCGAAAGGC TAATACAAATCCAGCATACCGTCGCGGAAGTGCATGCCCGCATAGGAATTCC GGTAGAAATTGTCGAGATGGGGTTTCGGGTGCTGAAAAAAATCCTCTATCCG GTGATCTTCTCTTCGGATTATTCCGCCGCAGAAAAACTTCAGGTCTACCATTT CTCGATTAACAGTATTGATATCGCGATGGAAGTGATGACTCGCGCGTTTACC TTTAGTGACAGTAGTGCCTCAAAGGAAGATGAAAACTATCGTATCTTCTCGT TACTGGAAAACGCCGAAGAAGAAAAAGAACGGCAAATAGCCTCAATACTTT CATGGGAAATAGATATTATCTATAAAATCCTGCTGGATTCTGATTTAGGCAG TAGTTTGCCTTTAAGCCAGGCTGATTTTGGCCTGTGGTTTAACCATAAAGGTC GACATTATTTTAGTGGTATTGCCGAAGTAGGCCATATCTCCCGTCTGATTCA GGATTTCGACGGTATTTTCAATCAAACCATGCGTAACACCAGAAATTTGAAT AACAGAAGTCTGCGGGTGAAATTTTTATTACAGATAAGAAATACCGTATCGC AAATTATTACCTTGCTGCGTGAATTGTTTGAAGAAGTATCGCGCCACGAAGT CGGTATGGATGTACTGACGAAATTACTTAACCGCCGTTTCCTACCGACTATC TTCAAACGCGAAATTGCCCATGCCAACCGGACCGGTACACCGCTGTCAGTGC TGATTATTGACGTTGATAAATTCAAAGAGATCAACGATACGTGGGGCCATAA CACTGGTGATGAAATTCTGCGTAAAGTCTCTCAGGCCTTTTATGACAACGTC CGCAGTAGTGATTATGTTTTCCGCTACGGGGGCGATGAATTTATCATTGTTTT GACTGAAGCTTCTGAAAACGAAACGTTACGTACCGCAGAACGTATTCGCAG TCGGGTGGAGAAAACCAAACTGAAAGCCGCAAACGGCGAAGATATTGCCCT CTCACTTTCCATCGGTGCCGCCATGTTTAATGGTCATCCTGACTATGAGCGCC TCATTCAAATAGCCGATGAAGCTCTGTATATCGCCAAAAGACGAGGTAGAA ACCGTGTTGAACTCTGGAAAGCCAGTCTTTAG |
| **AdrA(Sequence No. 5)** |
| ATGTTCCCAAAAATAATGAATGATGAAAACTTTTTCAAAAAAGCGGCGGCG CACGGGGAGGAACCTCCTTTAACTCCTCAAAACGAACATCAGCGGTCCGGG CTGCGCTTCGCCCGTCGCGTCAGACTACCCCGTGCGGTTGGCCTGGCTGGCA TGTTCTTACCGATTGCTTCAACGCTGGTTTCACACCCGCCGCCGGGCTGGTG GTGGCTGGTGTTGGTCGGCTGGGCGTTCGTCTGGCCGCATTTAGCCTGGCAG ATAGCGAGCAGGGCCGTCGATCCGCTTAGCCGGGAAATTTACAACTTAAAA ACCGATGCAGTATTAGCGGGAATGTGGGTAGGCGTAATGGGCGTAAACGTG CTGCCTTCCACCGCGATGTTGATGATTATGTGTCTGAATTTGATGGGGGCAG GCGGCCCCCGTCTGTTTGTCGCGGGTCTGGTGTTGATGGTGGTTTCCTGCCTT GTCACCCTCGAGCTGACGGGCATTACCGTGTCGTTCAATAGTGCGCCGCTGG AATGGTGGCTCTCCCTTCCCATTATTGTCATTTATCCTCTGCTGTTTGGCTGG GTCAGCTACCAGACGGCAACCAAACTGGCGGAACATAAACGCAGGTTGCAG GTCATGAGTACCCGCGACGGCATGACGGGCGTGTATAACCGACGTCATTGG GAAACTATGTTACGCAATGAATTTGATAACTGTCGGCGGCATAATCGCGATG CAACGTTACTGATTATCGATATCGACCATTTCAAGAGCATCAACGATACCTG GGGCCATGATGTGGGCGATGAAGCGATTGTGGCGCTTACCCGACAGTTACA AATTACCCTGCGCGGTAGCGATGTGATTGGTCGGTTTGGCGGCGATGAGTTT GCAGTAATCATGTCCGGTACGCCAGCTGAGAGCGCCATTACCGCCATGTTAC GGGTGCATGAAGGGCTAAATACATTACGTTTGCCGAATACGCCACAGGTAA CTTTACGGATTAGTGTGGGGGTTGCGCCGCTGAACCCACAAATGAGTCACTA TCGTGAGTGGTTGAAATCGGCAGATTTGGCGCTTTACAAAGCAAAGAAAGC CGGACGTAACCGCACCGAAGTGGCGGCCTGA |
| **ydeH(Sequence No. 6)** |
| ATGATCAAGAAGACAACGGAAATTGATGCCATCTTGTTAAATCTCAATAAGGC TATCGATGCCCACTACCAGTGGCTGGTGAGTATGTTTCACAGCGTGGTCGCGA GAGATGCCAGTAAGCCAGAAATAACGGATAACCATTCTTATGGACTGTGCCAG TTTGGTCGGTGGATTGATCATCTGGGGCCACTCGATAACGATGAATTACCTTAC GTTCGGCTAATGGATTCTGCCCATCAACATATGCATAACTGTGGTCGGGAATTA ATGCTGGCTATTGTTGAAAATCACTGGCAGGACGCGCATTTCGACGCCTTTCA GGAGGGGTTGCTTTCTTTTACTGCGGCATTAACCGATTACAAAATTTATTTGCT GACGATCCGTAGCAATATGGATGTTTTGACGGGATTGCCGGGTCGTCGGGTTC TTGATGAATCCTTTGATCATCAGTTACGCAACGCTGAGCCTCTGAATCTTTATT TAATGTTGTTGGATATTGACCGATTTAAATTGGTTAATGATACCTACGGGCATTT AATCGGCGATGTAGTATTACGCACCCTGGCAACTTACTTAGCCAGTTGGACGC GTGATTACGAAACGGTTTATCGCTACGGGGGCGAAGAATTTATCATTATTGTCA AAGCGGCTAATGATGAAGAAGCATGTCGTGCAGGTGTCAGAATTTGCCAGTT AGTCGATAACCATGCCATCACACATTCTGAAGGGCATATCAACATTACCGTGA CAGCAGGTGTGAGTCGCGCATTTCCTGAAGAGCCTCTGGATGTGGTCATTGG AAGAGCGGACCGGGCAATGTATGAGGGTAAGCAAACCGGAAGAAATCGCTG CATGTTTATTGACGAACAAAATGTGATTAACCGAGTTTAA |
| **YfiN(Sequence No. 7)** |
| ATGAATAAGGAATTTTCTCTGTCCAGGCCAACATTTAAACGCACACTACGGCG GATTAGTATAATCAGCGTGCTGCTTACAATGACATTGATCTGGCTATTAATTTGC GTTGCGTCTGTCCTTACGCTCAAACAGTATGCGCAAAAGAATCTCGATTTGAC CGCCGCCACAATGGCCCATAGCCTTGAAGCGGCACTGGTATTTTCCGATAACG CGGCCGCAGCGGAAACGCTCGCCACACTGGGACGCCAGGGACAATTTTCAG CGGCGGAGGTCCGCGATAAAAATGGCCGTACTATCGCCTCATGGCGCTATGAT GCGCGAGCCGCAGACGATAAGCTCATTGGCTTAATTAGCCACTGGCTTTTTCC ATTGCCGGTATCACAACCCGTCTGGCACAACGGCAGGGCCATCGGCGAAGTA CGGCTTGTCGCCCGCGACAGCCTTATTGGTCATTTTATCTGGCTATCGCTGGCA GTGCTGACAGGATGTATTCTGCTGGCATCCGGCATTGCCCTGCTGCTCACGCG TTATTTACACAATGGCGTTGTGGATGCGCTGCAAAATATTACTGAAGTTGTACA CGACGTTCGCACTAACCGAAATTTTTCACGCCGGGTACCTGATGAGCGTATTG CGGAATTTCACCTGTTTGCGCAGGATTTCAATAGCCTTCTGGATGAGATGGAA GAATGGCAGCTACGGCTTCAGGCTAAAAATGCCCAGTTACTACGTACCGCGTT GCACGATCCGCTGACGGGGCTTGCCAATCGCGCGGCATTTCGCAGCTGTATTA ACGCGCTGATGAAGGACAATTCCGCTCGTAGCAGTTCGGCATTGTTATTTCTG GATGGCGATAACTTTAAATATATTAATGATACCTGGGGACATGCGGCAGGCGA CCGCGTACTTATAGAGGTTGCCAAAAGATTAGCGGAATTCGGTGGTAGCCGTT ATCAGACTTACCGACTCGGCGGCGATGAATTTGCGATGGTGCTTTACGATGTA CATTCGGAATATGAAGTACAACGTATTTGCGCAGCGCTATCCCAGGCGTTTAAT CGACCTTTTGAACTGCATAACGGCCAGCGAATAACGATGACCCTGAGTATTGG CTTTGCGCTGACATGGGAACATGCCACTGCCGAAAAACTACAAGAACTGGCC GATCGAAATATGTATCAGGCTAAACACCGGCGTGCGGAGCGCTCGCTAAACTA A |
| **Adra(Sequence No. 8)** |
| ATGTTCCCAAAAATAATGAATGATGAAAATTTTTACCGAAAAGCGGTTGAAC AGGCGGTAGCGCCGCCTGACCCTCCTGATGATCGTCAGCGCTCCGGGTTGCG CTTCGCCCGGCGCATCAGGCTACCGCGTGCGGTGGGGTTAGGGGGGATGTTT TTACCGGTGGCCGCCGTGTTAGTGACGCAACCTGTCTTTGGCGGCTGGTGGC TATTGCTGGTCGGCTGGTCTTTTGTCTGGCCGCATCTGGCATGGCAGTGGGC GGCGAAAGCCCTTGATCCGCTACGCCAGGAAATTTACAACCTGAAAGTGGA TGCGATCCTCTCCGGCATGTGGATCGCGCTGATGGGCGTCAATATGTTGCCT GCCGCCGCGCTGTTTATGATGATGAGCATGAATCTGATGGGCGCTGGCGGGC GGCGGCTATTCACTGTCGGAATGGGCCTTTTGCTGGCGTCATGTCTGGTGAC GTTGCAGCTTGCCGGGCTACCCGTCGCGATGCGAAGCTCGTCGCTGGAAGTC ACGCTCTCTTTGCCCGTCATTATGTTGTATCCGCTTCTGTTCGCCTGGGTAAG TTACCAAACGGCCATTAAATTAGCGGAACATAAGCGACGCTTGCAGGCGAT GAGCTCGCGGGATGGCATGACCGGCGTCTATAACCGACGCCACTGGGAAAT TTTATTACGTAATGAATTCGATCACAGCCGACGCCACCATCGGGAGGCGACA TTGTTGATTATCGATATCGATCATTTTAAGAGCATTAACGACACCTGGGGAC ATGATGTTGGGGATGAGGCTATTATCGCCCTGACGCGTCAATTACAGATAAC GCTACGCGGGAGTGATATTATCGGGCGCTTTGGCGGCGACGAATTTGCGGTG ATTATGTGCGGAACGCCGGCAGACAGCGCGATTACCGCCATGTCGCGAGTG CATGAACGGCTAAATACCTTACGCTTGCCGGGCGCGCCGCAGGTCATGTTAC GTATCAGCGTGGGCGTGGCGCCGTTAACGCCACAAATCGGTCATTATCGGGA ATGGCTAAAGTCTGCGGATATGGCGCTTTACAAAGCAAAGAATGCCGGACG TAACCGCACCGAAGTGGCGGCATGA |
| **DosC(S)^{*}(Sequence No. 9)** |
| ATGAATTTGCATCATAAAGCGCTCAGGCACTTTATCTCGGCAAGCGTCATCGT TTTGACATCGTCCTTCCTGATTTACGAACTTATTGCCAGCGACAGGGCAATGA ATGCCTATATGCGTTATATCATGGAGAGAGCCGATTCGTCGTTTTTGTACGATA AGTACCAGAATCAGAGCATCGCCGCACATTTGATGCGAACTTTTGAAGCGCC GGGAGACCCCGTCACCGCAGAAAAACACCGCGCGTTTTGCGACGCCTTTGA AGCTATTAACGGTACGCACGGTGTCAACCTGACCCGGCATAATTATCCGGCGC TACATGGTACTCTGCAAACCGCCGCTACACAGTGTACCGATAATCTTGATGAC GCCCTTTTACTGCCTGCATTTGATCAGGCGGTAAGCATCAACCGTTCGCAGGA CGACCATAGTCACGGGCTGGGCACACTGGAGCTTAAATTCCGTTATTACGTTG ATTTAAATAAACATTATGTCTATTTCTATGATTTAATCAACTCACGGCGCTTCGC CATGCATCGTTGGACTTTTTTACAAAAAGGCACAATGGGTATTAACAGAAAAG ATATAGATAAACTTTTTACCGGCCGTACGGTTATTTCAAGTATCTACATGGATG ATATTACTCAGGAAAACGTCATGAGTTTTTTAACGCCAGTCTATCTGGCGGGA TCGTTAAAAGGCATCGTGATGGTGGATGTTAACCAGGATAATTTAAAAAATATT TTTTATACCCAGGACCGTCCGCTGGTTTGGCGTTATCTTAACGTAACACTAAA GGATATGGACTCCGGAAAAGAAATTATTATTAATCAAAGCAAAAATAATCTGT TTCAATATGTGAATTATAGCCATGATATCCCTGGTGGGCTGCGCGTTTCGTTGT CCCTTGATTTAACCTATTTCCTTGTCTCGTCCTGGAAAGCGCTGGCCTTTTACT TACTGGCAACGGCGCTCCTGCTTAATATGGTACGGATGCACTTTCGGCTTTATC GCAACGTCACACGCGAAAATATTAGCGATGCCATGACCGGGCTTTACAACCGT AAAATATTAACACCGGTGCTGGAACAGCGACTGCAACGCCTGGTCAATACCG GGACGCCGGTGACATTTGTCGCTATTGATTGCGATAGGTTAAAACTGATCAAC GATACCCAGGGGCACCAGGAAGGCGACCGAATTATAACCCTGTTGGCGAAAG CGATTAAAACATCGATTCGTAAAAGCGATTACGCCATTCGCCTCGGCGGCGAT GAGTTCTGTATTATTCTTGTTGATTACGCGGCGGATTTGGCTATCCATCTGCCG GAGCGTATTATTCGTAACCTGCAAATTATCGCACCGGATAAGACAGTCCATTTT TCTGCCGGGATTTATAATATGCAGCCCAATGATACGATTAATGATGCCTACCAG GCTTCCGATGCGCAGCTCTATCTGAACAAACAACAAAAACAACATCGTTCATC ATAG |
| **CGAS(Sequence No. 10)** |
| ATGCAGCCTTGGCACGGAAAGGCCATGCAGAGAGCTTCCGAGGCCGGAGCC ACTGCCCCCAAGGCTTCCGCACGGAATGCCAGGGGCGCCCCGATGGATCCCA CCGAGTCTCCGGCTGCCCCCGAGGCCGCCCTGCCTAAGGCGGGAAAGTTCGG CCCCGCCAGGAAGTCGGGATCCCGGCAGAAAAAGAGCGCCCCGGACACCCA GGAGAGGCCGCCCGTCCGCGCAACTGGGGCCCGCGCCAAAAAGGCCCCTCA GCGCGCCCAGGACACGCAGCCGTCTGACGCCACCAGCGCCCCTGGGGCAGA GGGGCTGGAGCCTCCTGCGGCTCGGGAGCCGGCTCTTTCCAGGGCTGGTTCT TGCCGCCAGAGGGGCGCGCGCTGCTCCACGAAGCCAAGACCTCCGCCCGGG CCCTGGGACGTGCCCAGCCCCGGCCTGCCGGTCTCGGCCCCCATTCTCGTAC GGAGGGATGCGGCGCCTGGGGCCTCGAAGCTCCGGGCGGTTTTGGAGAAGT TGAAGCTCAGCCGCGATGATATCTCCACGGCGGCGGGGATGGTGAAAGGGGT TGTGGACCACCTGCTGCTCAGACTGAAGTGCGACTCCGCGTTCAGAGGCGTC GGGCTGCTGAACACCGGGAGCTACTATGAGCACGTGAAGATTTCTGCACCTA ATGAATTTGATGTCATGTTTAAACTGGAAGTCCCCAGAATTCAACTAGAAGAA TATTCCAACACTCGTGCATATTACTTTGTGAAATTTAAAAGAAATCCGAAAGA AAATCCTCTGAGTCAGTTTTTAGAAGGTGAAATATTATCAGCTTCTAAGATGCT GTCAAAGTTTAGGAAAATCATTAAGGAAGAAATTAACGACATTAAAGATACA GATGTCATCATGAAGAGGAAAAGAGGAGGGAGCCCTGCTGTAACACTTCTTA TTAGTGAAAAAATATCTGTGGATATAACCCTGGCTTTGGAATCAAAAAGTAGC TGGCCTGCTAGCACCCAAGAAGGCCTGCGCATTCAAAACTGGCTTTCAGCAA AAGTTAGGAAGCAACTACGACTAAAGCCATTTTACCTTGTACCCAAGCATGCA AAGGAAGGAAATGGTTTCCAAGAAGAAACATGGCGGCTATCCTTCTCTCACA TCGAAAAGGAAATTTTGAACAATCATGGAAAATCTAAAACGTGCTGTGAAAA CAAAGAAGAGAAATGTTGCAGGAAAGATTGTTTAAAACTAATGAAATACCTT TTAGAACAGCTGAAAGAAAGGTTTAAAGACAAAAAACATCTGGATAAATTCT CTTCTTATCATGTGAAAACTGCCTTCTTTCACGTATGTACCCAGAACCCTCAAG ACAGTCAGTGGGACCGCAAAGACCTGGGCCTCTGCTTTGATAACTGCGTGAC ATACTTTCTTCAGTGCCTCAGGACAGAAAAACTTGAGAATTATTTTATTCCTGA ATTCAATCTATTCTCTAGCAACTTAATTGACAAAAGAAGTAAAGAATTTCTGA CAAAGCAAATTGAATATGAAAGAAACAATGAGTTTCCAGTTTTTGATGAATTT TGA |
| **Cgas(Sequence No. 11)** |
| ATGGAAGATCCGCGTAGAAGGACGACGGCGCCACGCGCTAAGAAGCCGTCC GCGAAGCGCGCCCCGACGCAGCCCAGCAGGACCAGGGCCCACGCGGAAAG CTGCGGCCCGCAAAGGGGGGCTCGATCGCGGCGGGCGGAGCGTGACGGGGA CACCACGGAGAAGCCACGTGCCCCAGGGCCCCGAGTGCATCCAGCAAGGGC CACTGAGCTCACCAAAGATGCACAGCCCTCGGCCATGGACGCGGCAGGAGC CACCGCGCGGCCTGCCGTCCGGGTGCCCCAGCAGCAGGCCATCCTGGATCCG GAGCTGCCCGCCGTACGGGAGCCCCAGCCGCCCGCGGATCCCGAGGCGCGG AAAGTCGTAAGGGGACCTAGCCACAGAAGGGGCGCGCGCTCCACCGGGCAG CCCAGAGCGCCGCGAGGGTCCAGGAAGGAACCGGACAAGCTAAAGAAGGT GCTGGACAAATTGAGATTGAAACGCAAAGATATCTCGGAGGCGGCCGAGACG GTGAATAAAGTTGTGGAACGCCTGCTGCGCAGAATGCAGAAACGGGAGTCG GAGTTCAAAGGTGTGGAGCAGCTGAACACTGGCAGCTACTATGAACATGTGA AGATTTCTGCTCCTAATGAATTTGATGTTATGTTTAAACTGGAAGTCCCCAGGA TTGAGCTACAAGAATATTATGAAACAGGTGCTTTCTATCTTGTGAAATTCAAA AGAATTCCACGAGGAAATCCGCTGAGTCATTTCTTAGAAGGGGAAGTATTATC AGCTACCAAGATGCTGTCAAAGTTTAGGAAAATCATTAAAGAAGAAGTTAAA GAAATCAAAGATATAGATGTCAGTGTGGAGAAGGAAAAACCAGGAAGCCCT GCTGTAACACTTCTTATCAGGAACCCTGAAGAAATCTCTGTGGATATAATTCTG GCTTTGGAGTCAAAAGGCAGCTGGCCTATTAGTACCAAAGAAGGACTACCTA TTCAAGGCTGGCTGGGCACAAAAGTGAGGACCAATCTAAGAGCGAGAGCCG TTTTATCTCGTACCCAAGAATGCAAAGGATGGAAATAG |
| *(S) indicates a sequence derived from the *Salmonella* genus. |
| Sequences without a separate designation are derived from *Escherichia coli.* |

**[Table 2]**

| Primer identification code | Sequence No. | Sequence |
|---|---|---|
| pJH18-DisA-F | Sequence No. 12 | AAAGGCCTAAAGAGGAGAAAGCTAGC ATGGAAAAAG |
| pJH18-DisA-R | Sequence No. 13 | GGACTAGTTTAGTGATGGTGATGGTGAT GCAGTTGTCTGTCTAAATAATGCTTCTC TT |
| pJH18-CdaA-F | Sequence No. 14 | AAAGGCCTAAAGAGGAGAAAGCTAGC ATGGCTTT |
| pJH18-CdaA-R | Sequence No. 15 | GGACTAGTTTAGTGATGGTGATGGTGAT GTCCATTTT |
| pJH18-CdaS-F | Sequence No. 16 | AAAGGCCTAAAGAGGAGAAAGCTAGC ATGAAGGCTATGCGCTATGAGCAGATAT C |
| pJH18-CdaS-R | Sequence No. 17 | GGCCGACGTCGACTTACGTTCTTGGTG AAATTAACGGATATAATAC |
| pJH18-DosC-F | Sequence No. 18 | AAACTAGTAAAGAGGAGAAAGCTAGC ATGGAGATGTATTTTAAAAGAATGAAA GATGAGTG |
| pJH18-DosC-R | Sequence No. 19 | GGGAGCTCTTAGTGATGGTGATGGTGA TGAAGACTGGCTTTCCAGAGTTCAACA |
| pJH18-AdrA-F | Sequence No. 20 | AAAGGCCTAAAGAGGAGAAAGCTAGC ATGTTCCCAAAAATAATGAATGATGAA AACTTTTTCAAAA |
| pJH18-AdrA-R | Sequence No. 21 | GGACTAGTTTAGTGATGGTGATGGTGAT GGGCCGCCACTTCGGTGC |
| pJH18-ydeH-F | Sequence No. 22 | AAAGGCCTAAAGAGGAGAAAGCTAGC ATGATCAAGAAGACAACGGAAATTGAT GC |
| pJH18-ydeH-R | Sequence No. 23 | GGACTAGTTTAGTGATGGTGATGGTGAT GAACTCGGTTAATCACATTTTGTTCGTC AATAA |
| pJH18-yfiN-F | Sequence No. 24 | AAAGGCCTAAAGAGGAGAAAGCTAGC ATGAATAAGGAATTTTCTCTGTCCAGGC |
| pJH18-yfiN-R | Sequence No. 25 | GGACTAGTTTAGTGATGGTGATGGTGAT GGTTTAGCGAGCGCTCCGCA |
| pJH18-Adra-F | Sequence No. 26 | AAAGGCCTAAAGAGGAGAAAGCTAGC ATGTTCCCAAAAATAATGAATGATGAA AATTTTTACC |
| pJH18-Adra-R | Sequence No. 27 | GGACTAGTTTAGTGATGGTGATGGTGAT GTGCCGCCACTTCGGTGCG |
| pJH18-DosC(S)-F | Sequence No. 28 | AAAGGCCTAAAGAGGAGAAAGCTAGC ATGAATTTGCATCATAAAGCGCTCAGG |
| pJH18-DosC(S)-R | Sequence No. 29 | GGACTAGTTTAGTGATGGTGATGGTGAT GTGATGAACGATGTTGTTTTTGTTGTTT GTTCA |
| pJH18-CGAS-F | Sequence No. 30 | AAACTAGTAAAGAGGAGAAAGCTAGC ATGCAGCCTTGGCACGGAAAG |
| pJH18-CGAS-R | Sequence No. 31 | GGGAGCTCTTAGTGATGGTGATGGTGA TGAAATTCATCAAAAACTGGAAACTCA TTGTTT |
| pJH18-Cgas-F | Sequence No. 32 | AAAGGCCTAAAGAGGAGAAAGCTAGC ATGGAAGATCCGCGTAGAAGGACGA |
| pJH18-Cgas-R | Sequence No. 33 | GGGAGCTCTTAGTGATGGTGATGGTGA TGTTTCCATCCTTTGCATTCTTGGGTAC G |

### Example 2. Confirmation of anticancer effects of a DNA construct expressing a STING agonist synthase

### [2-1] Construction of recombinant strains and confirmation of STING agonist production

The DNA construct (plasmid) prepared in Example 1 was introduced, by electroporation, into SHJ2037 (relA::cat, spoT::kan, SLppGpp), CNC18 (ΔrelA, ΔspoT, ΔSPI1, ΔSPI2), VNP20009, *E. coli* DH5α, and *E. coli* Nissle, which are ppGpp-deficient *Salmonella typhimurium* (S. typhimurium) strains, and then cultured overnight using LB medium containing 100 µg/ml ampicillin. On the following day, the culture was diluted at a ratio of 1:100 using fresh LB medium containing ampicillin, and when the OD600 reached 0.5 to 0.7 through additional culture, doxycycline diluted in ethanol was added to the culture to final concentrations of 0, 10, 50, 100, 300, and 500 ng/ml, followed by incubation in a shaking incubator at 200 rpm and 37°C.

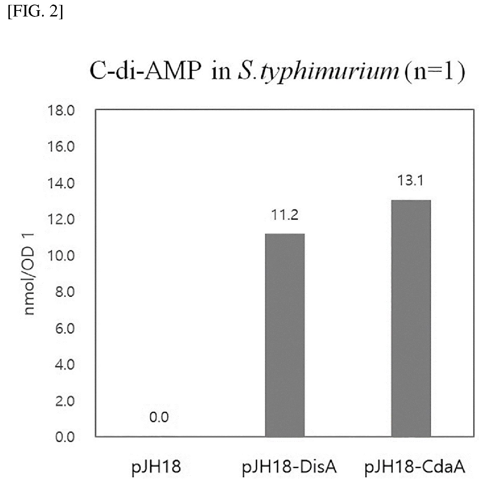

Among the culture media of the strains, C-di-AMP was first measured by liquid chromatography, and as a result, it was confirmed that C-di-AMP was successfully produced from the recombinant strains (Fig. 2).

### [2-2] Confirmation of immune-stimulating effects of transformed recombinant strains

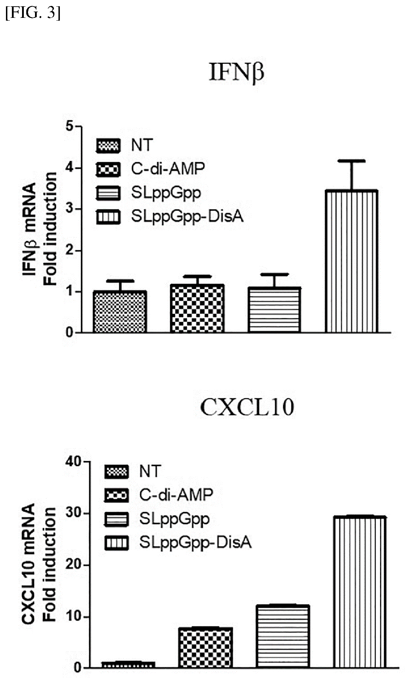

RAW 264.7 cells, a macrophage cell line, were treated with 5 nM/well of C-di-AMP, a *Salmonella* strain transformed with an empty vector (SLppGpp), or a *Salmonella* strain transformed with the vector prepared in Example 1 (SLppGpp_pJH18-DisA), followed by additional incubation for 9 hours. Thereafter, expression of IFNβ (Interferon β) and CXCL10 (C-X-C motif chemokine 10) was measured from the culture medium. As a result, when the *Salmonella* strain transformed with a DNA construct expressing a C-di-AMP synthase (SLppGpp_pJH18-DisA) was administered, it was confirmed that the immune-stimulating effect was remarkably increased (Fig. 3).

### [2-3] Confirmation of anticancer effects of transformed recombinant strains

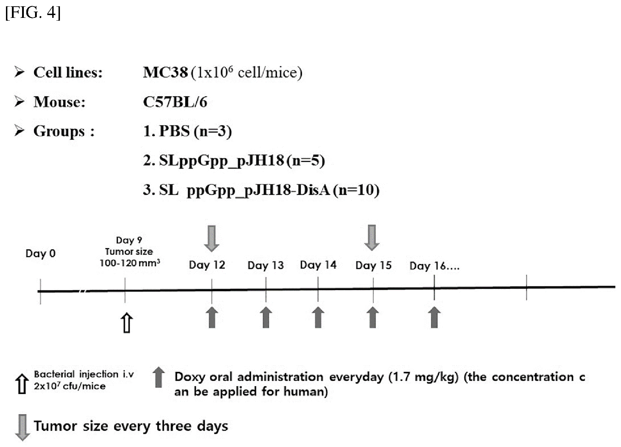

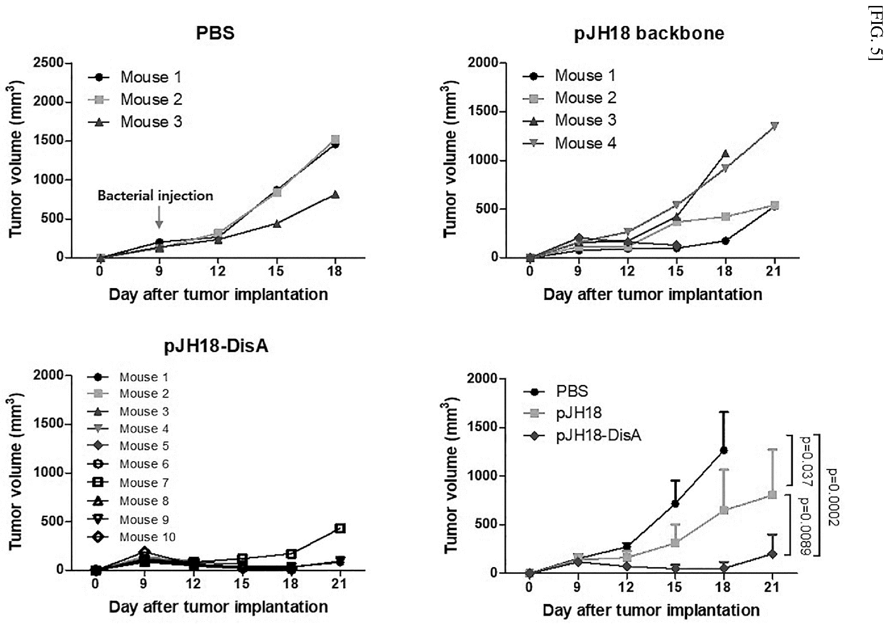

In order to confirm whether recombinant strains having a DNA construct expressing a STING agonist synthase induce anticancer effects in living animal subjects, C57BL/6 mice (Orient Company, Korea) aged 5 to 6 weeks and weighing 20 to 30 g were administered cancer cells, and when the tumor size reached about 100 mm³, a *Salmonella* strain (SLppGpp_pJH18-DisA) transformed with the DNA construct expressing a C-di-AMP synthase was injected via the tail vein at a dose of 2×10⁷ cfu/mice. For imaging and evaluation of tumor size in the tumor animal model, 2% isoflurane was used for anesthesia, and during surgery, 200 mg/kg of ketamine and 10 mg/kg of xylazine were used. In order to induce expression of C-di-AMP, doxycycline was orally administered daily at a dose of 1.7 mg/kg, and tumor size was measured at intervals of 3 days. Evaluation of tumor size (mm³) was performed using (length × height × width)/2, and when the tumor size of the animal model exceeded 1500 mm³, the animal model was euthanized (Fig. 4). As a result of the above experiment, it was confirmed that the pJH18-DisA strain exhibited a very excellent tumor suppression effect (Fig. 5).

### [2-4] Identification of issues with the pJH18-DisA strain

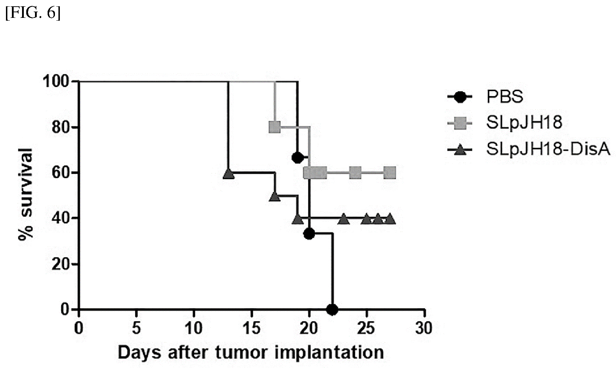

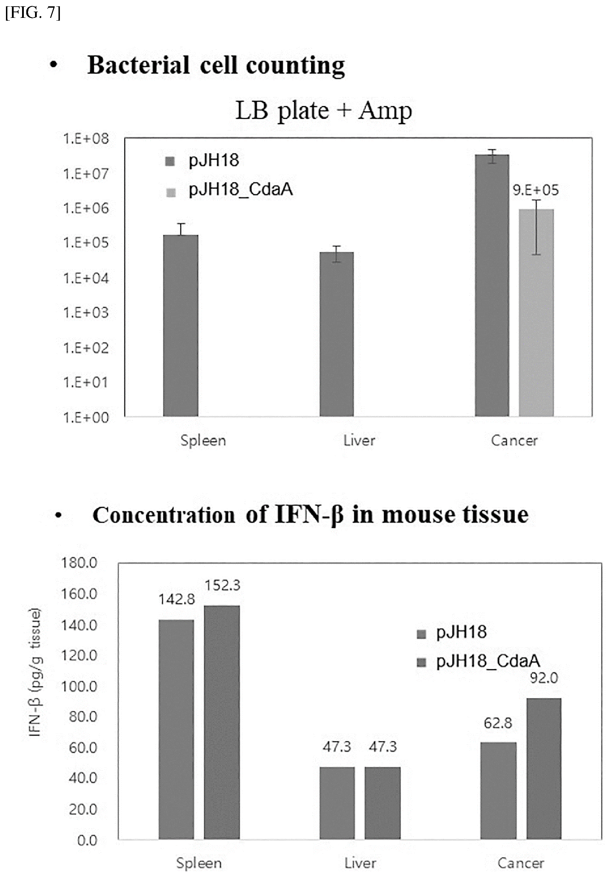

Although Example 2-3 demonstrated excellent anticancer effects of the *Salmonella* strain transformed with a DNA construct expressing a C-di-AMP synthase, it also provided clues to unexpected risk factors. Some of the mice administered with the pJH18-DisA strain died suddenly one day after doxycycline administration, and autopsy revealed thrombosis along with renal enlargement (Fig. 6). In addition, the number of bacteria (*Salmonella*) in the tumors decreased rapidly by about 100-fold compared to before expression of the C-di-AMP synthase (= before administration of 0.5 µg/ml doxycycline), and the expression level of IFN-β in the tumors increased explosively (Fig. 7), suggesting a possible cytokine storm.

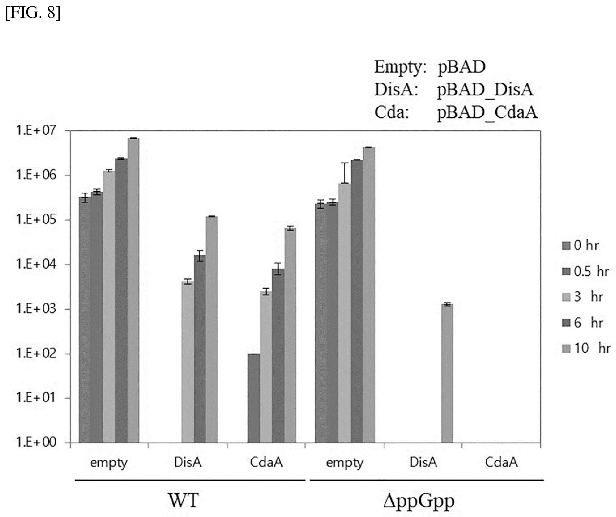

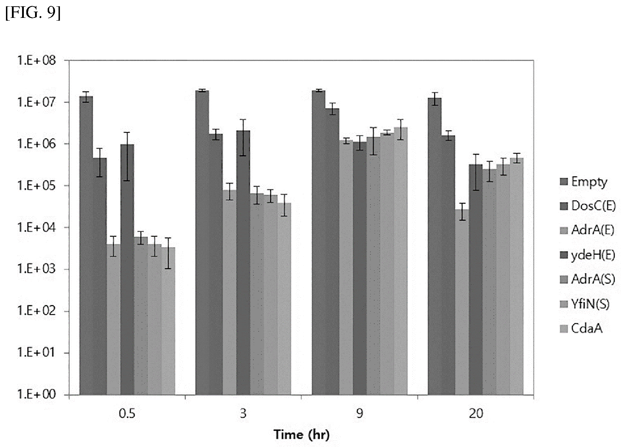

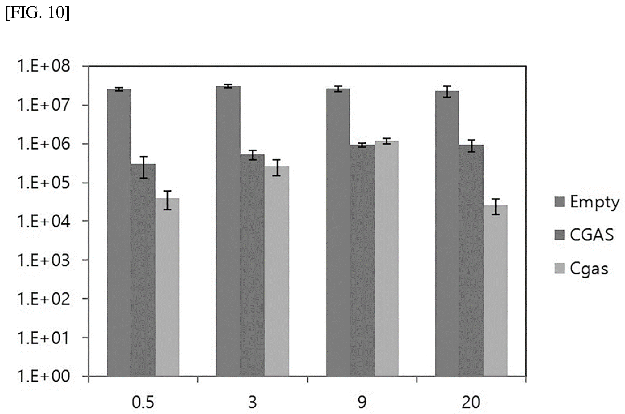

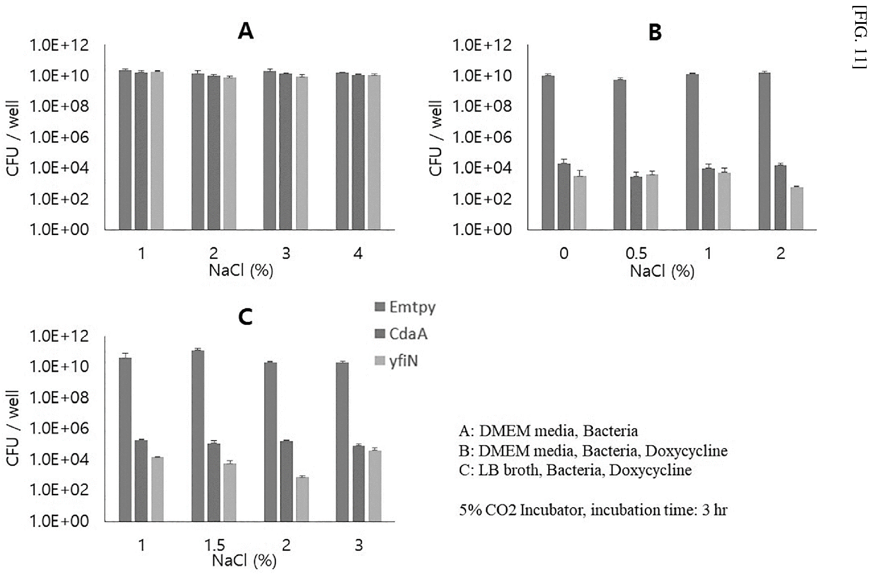

To clearly verify the hypothesis, ppGpp-deficient *Salmonella* strains (SLppGpp) or wild type (WT) *Salmonella* strains transformed with DisA or CdaA genes capable of expressing C-di-AMP were prepared, and each strain (3×10⁵ cells/well) was cultured in vitro in DMEM animal cell culture medium (with 10% FBS). As a result, when *Salmonella* strains expressing a C-di-AMP synthase gene were cultured in the animal cell culture medium, it was confirmed that the number of bacteria rapidly decreased (Fig. 8). Additional studies showed that this phenomenon similarly occurred in strains expressing not only C-di-AMP but also C-di-GMP and cGAMP synthases (Figs. 9 and 10). In order to confirm whether this phenomenon was affected by osmotic pressure of the culture medium, strains were cultured under varying salt concentrations, and as a result, it was observed that the strains did not grow regardless of osmotic concentration (Fig. 11). Furthermore, even in experiments using LB medium, which is a bacterial-specific medium, the number of strains rapidly decreased, indicating that the issue was not due to the medium components.

Artificial introduction of a STING agonist synthase gene into recombinant strains results in regulation of various physiological processes, such as maintenance of cell wall homeostasis, DNA repair, regulation of gene expression, and production of virulence factors, by the STING agonist within the strain. However, when the intracellular concentration of the STING agonist is not properly regulated and accumulates excessively, it may negatively affect the growth of Gram-negative bacteria.

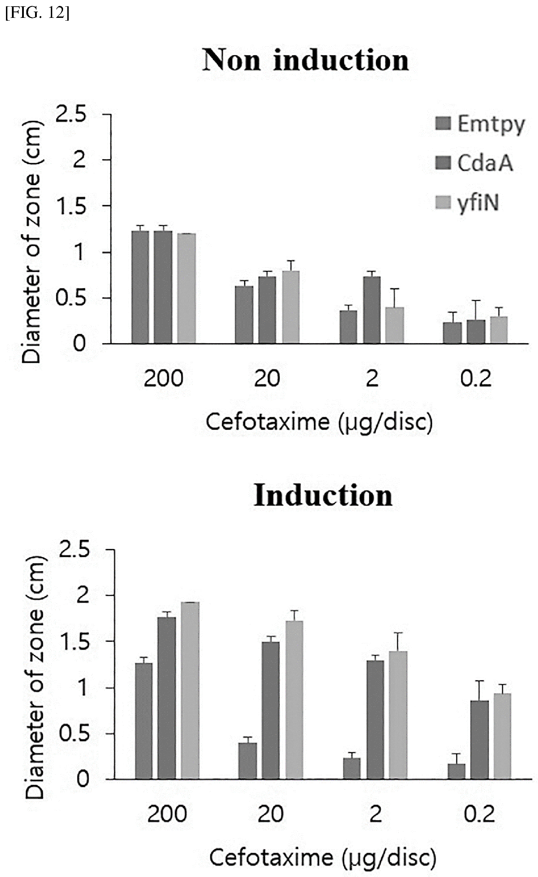

When STING agonists are excessively accumulated in bacteria, cell wall synthesis is inhibited, leading to defects in the cell wall, which may result in cell lysis and death. To confirm this, strains were cultured in media treated with varying concentrations of cefotaxime, an antibiotic that inhibits bacterial cell wall synthesis, and as a result, it was confirmed that bacteria did not grow simultaneously with expression of the STING agonist synthase (= administration of doxycycline) (Fig. 12 and Table 3).

**[Table 3]**

| | Emtpy | CdaA | yfiN | Cgas |
|---|---|---|---|---|
| VNP20009 | 0.37 | 0.70 | 0.83 | 0.90 |
| SHJ2037 | 0.37 | 1.30 | 1.60 | 1.13 |
| CNC18 | 0.47 | 1.53 | 1.77 | 0.97 |
| DH5a | 0.37 | 0.73 | 0.73 | 0.83 |
| Nissle | 0.37 | 0.63 | 0.80 | 0.63 |

In order to use recombinant strains as anticancer therapeutic agents, growth of the strains within tumors is required; however, expression of the STING agonist synthase was determined to be a growth-inhibiting factor, such as weakening of the cell wall within the strain. Taken together, in recombinant strains into which a gene encoding a STING agonist synthase is introduced, the endogenous STING agonist synthase should be synthesized within the bacterial cells and retained intracellularly; however, expression of the STING agonist synthase gene causes intracellular signaling imbalance, resulting in dissolution of the cell wall (bacterial lysis). This exposes cancer-bearing subjects (for example, tumor animal models) to excessive amounts of STING agonist synthase and bacterial lysates in the short term, thereby inducing a cytokine storm in the immune system of the subject.

### [2-5] Utilization as a bacterial lysis system

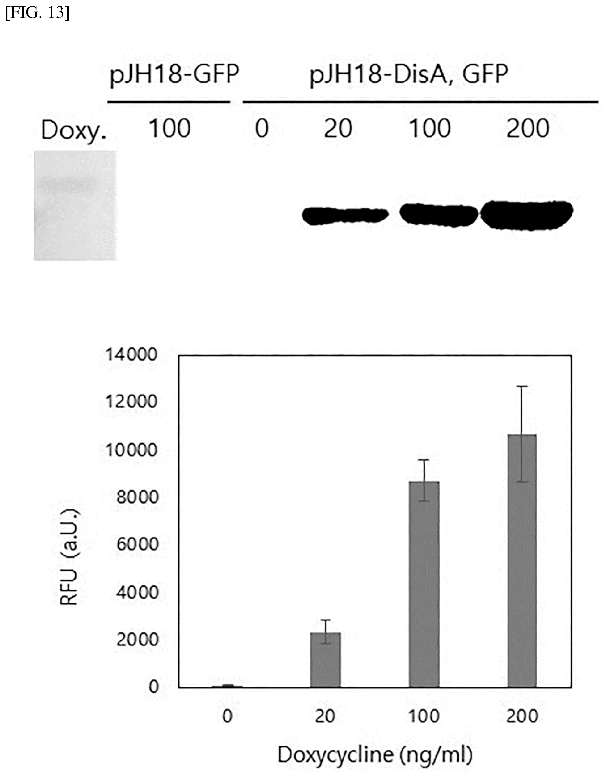

Since expression of the STING agonist synthase gene in bacteria interferes with cell growth or causes lysis, in order to confirm the potential of using this as a system for releasing and delivering foreign proteins or nucleic acid molecules that cannot be secreted from bacteria, a GFP fluorescent gene was introduced into the SLppGpp strain using the pJH18-DisA plasmid. Thereafter, doxycycline diluted in ethanol was added to the culture so that the final concentration of the culture medium became 0, 20, 100, and 200 ng/ml, followed by culturing in a shaking incubator at 200 rpm and 37°C. After inducing expression of DisA and GFP with doxycycline, the supernatant was collected and activity was confirmed by western blotting and a fluorescence reader. As a result, it was confirmed that GFP, which was not secreted when expressed alone, was released into the medium upon expression of DisA (Fig. 13). These results suggest that when the STING agonist synthase is expressed only within the strain, it can be used as a novel bacterial lysis protein.

### Example 3. Preparation of an improved DNA construct expressing a STING agonist synthase

In order to maintain the effects of Example 2 while improving the problems, a signal sequence was additionally introduced into the DNA construct prepared in Example 1 (Table 4).

**[Table 4]**

| Signal sequence identification code | Sequence No. | Sequence |
|---|---|---|
| ArgT | Sequence No. 34 | ATGAAGAAGACCGTTCTCGCTTT GTCTTTGCTGATAGGTCTGGGCG CGACGGCGGCCAGTTACGCC |
| AroQ | Sequence No. 35 | ATGATTCGTCATATCGCCATTTTT CTTTGTTCTTTATTGATGTGCAGC ACCACTTTTGCC |
| FlgH | Sequence No. 36 | ATGCAAAAATACGCGCTTCACGC TTACCCAGTTATGGCCCTGATGG TCGCGACGCTGACAGGA |
| FlgI | Sequence No. 37 | ATGGTGTTTAAAGCTCTTGCAGG AATCGTTCTGGCACTGGTTGCCA CTCTGGCGCACGCC |
| HiuH | Sequence No. 38 | ATGAAACGACATATACTGGCTAC CGTGATAGCATCTCTTGTTGCAG CCCCGGCAATGGCGCTGGCC |
| Lpp2 | Sequence No. 39 | ATGAACCGTACTAACCAGCTGAT CTTAGGTGCAGTAGTTCTGGGTT CCACGTTACTGGCAGGT |
| MltF | Sequence No. 40 | ATGTTGAAAAAATTAAAGATTAA TTATCTGTTCATCGGCATATTGAC GCTGCTGCTGGCAGCAGCCCTTT GGCCCTCAATCCCCTGGTTC |
| OmpA | Sequence No. 41 | ATGAAAAAGACAGCTATCGCGAT TGCAGTGGCACTGGCTGGTTTCG CTACCGTAGCGCAGGCC |
| OmpD | Sequence No. 42 | ATGAAACTTAAGTTAGTGGCAGT GGCAGTGACTTCCCTGTTGGCAG CAGGCGTTGTAAATGCA |
| OmpF | Sequence No. 43 | ATGATGAAGCGCAAAATCCTGGC AGCGGTGATCCCTGCCCTGCTGG CTGCTGCAACCGCAAACGCA |
| PelB | Sequence No. 44 | ATGAAATACCTGCTGCCGACCGC TGCTGCTGGTCTGCTGCTCCTCG CTGCCCAGCCGGCGATGGCC |
| ScrY | Sequence No. 45 | ATGTACAGAAAAAGCACACTTG CGATGCTTATCGCTTTGCTAACC AGCGCTGCCTCAGCCCATGCG |
| SodC1 | Sequence No. 46 | ATGAAATACACAATATTGTCGCT GGTAGCTGGTGCGCTCATCAGTT GTTCAGCAATGGCA |
| Tsx | Sequence No. 47 | ATGAAAAAAACTTTACTCGCAGT CAGCGCAGCGCTGGCGCTCACC TCATCTTTTACTGCTAACGCA |
| UshA | Sequence No. 48 | ATGAAATTTTTGAAACGGGGTGT GGCGCTGGCGTTACTGGCGGCG TTCGCGCTGACGACTCAGCCTGC ACAGGCT |

### Example 4. Confirmation of anticancer effects of an improved DNA construct expressing a STING agonist synthase

### [4-1] Preparation of recombinant strains transformed with the improved DNA construct

The DNA construct (plasmid) prepared in Example 3 was introduced, by electroporation, into SHJ2037 (relA::cat, spoT::kan, SLppGpp), CNC18 (ΔrelA, ΔspoT, ΔSPI1, ΔSPI2), VNP20009, *E. coli* DH5α, and *E. coli* Nissle, which are ppGpp-deficient *Salmonella typhimurium* (*S. typhimurium*) strains, followed by overnight culture using LB medium containing 100 µg/ml ampicillin. Thereafter, the culture was diluted at a ratio of 1:100 using fresh LB medium containing ampicillin, and when the OD600 reached 0.5 to 0.7 through additional culture, doxycycline diluted in ethanol was added to the culture to final concentrations of 0, 10, 50, 100, 300, and 500 ng/ml, followed by culturing in a shaking incubator at 200 rpm and 37°C.

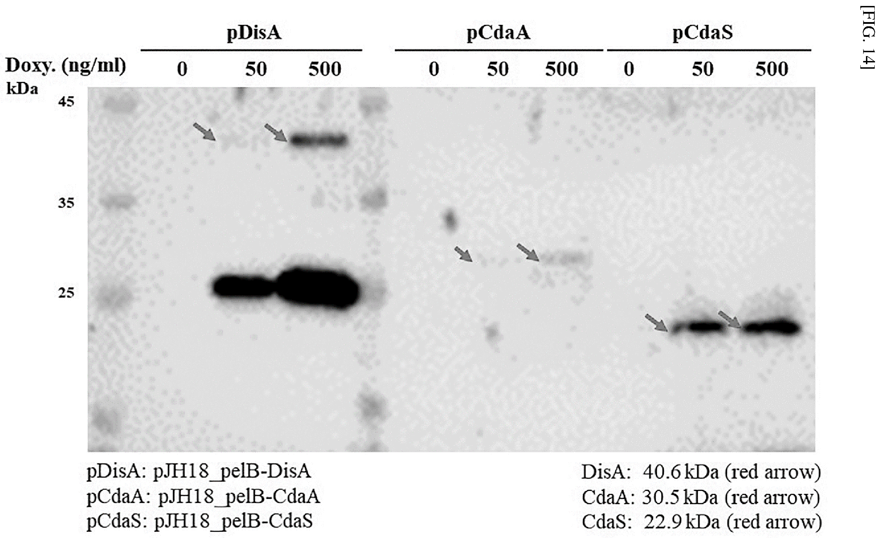

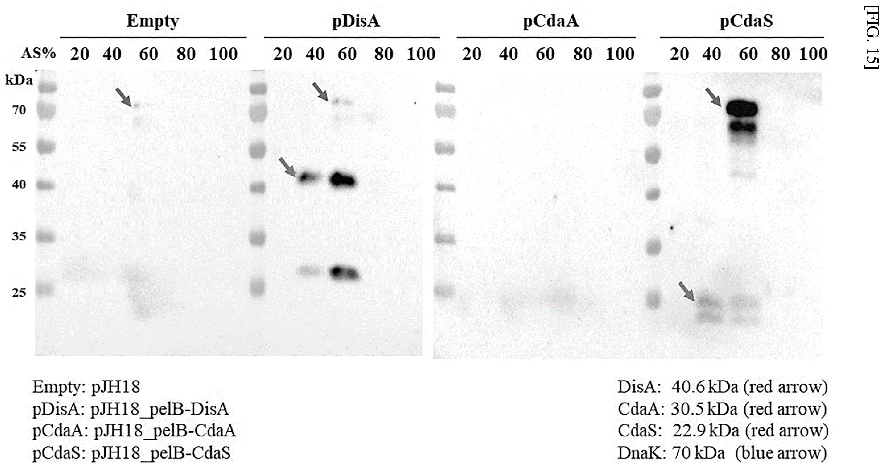

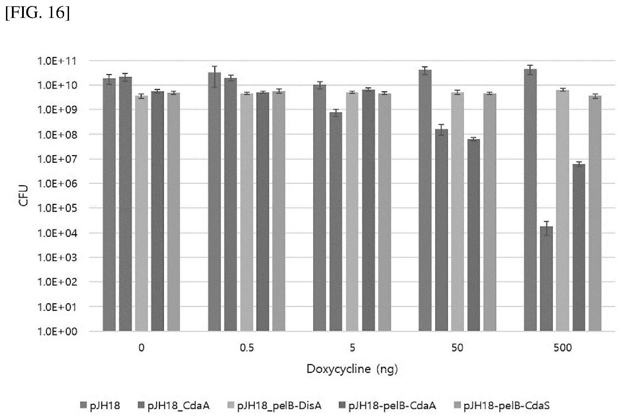

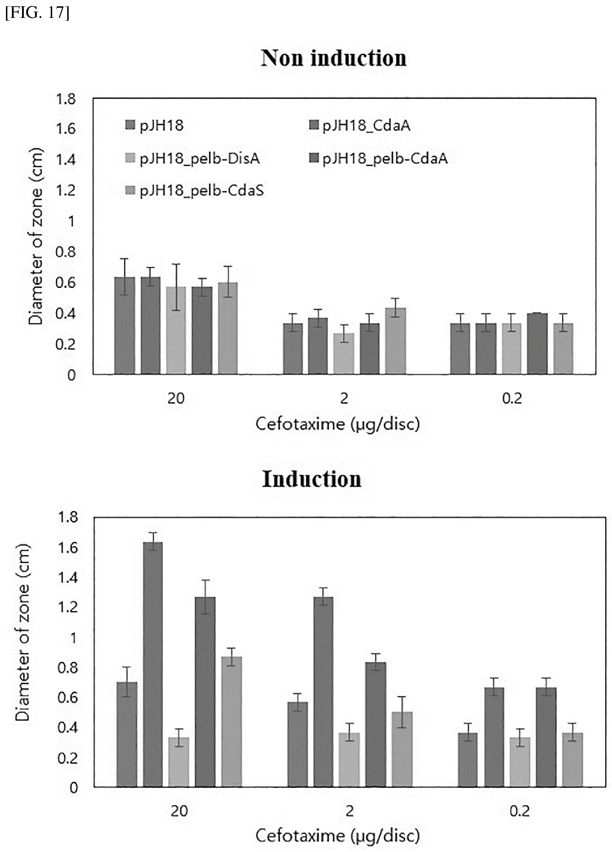

In culture media of strains into which DisA, CdaA, or CdaS was introduced together with pelB, the C-di-AMP synthase was confirmed by western blotting (SDS-PAGE 10%, Myc tag antibody). As a result, in strains into which DisA or CdaS genes were introduced, the C-di-AMP synthase was well secreted, whereas in strains into which the CdaA gene was introduced, secretion of the C-di-AMP synthase was not smooth (Fig. 14). In order to clearly confirm that the C-di-AMP synthase was secreted outside the bacterial cells without lysis of the cells, it was analyzed together with DnaK, an endogenous protein. As a result, in the strain into which the DisA gene was introduced, only the C-di-AMP synthase was selectively secreted outside the bacterial cells while endogenous proteins were well maintained inside the cells, whereas in the strain into which the CdaS gene was introduced, excessive detection of the endogenous protein DnaK was observed, indicating that such selectivity was significantly reduced (Fig. 15). In addition, when the strains were cultured while administering doxycycline, the strains into which DisA or CdaS genes were introduced were found to specifically maintain a state in which the bacterial cells were not lysed (Fig. 16). Furthermore, when the strains were cultured in media treated with cefotaxime at various concentrations, it was confirmed that antibiotic sensitivity decreased to the extent that cell lysis was prevented (Fig. 17). Taken together, when the STING agonist synthase was introduced together with a signal sequence, it had the effect of secreting the STING agonist synthase outside the bacterial cells while preventing lysis of the bacterial cells.

### [4-2] Evaluation of antibiotic sensitivity of recombinant strains transformed with the improved DNA construct

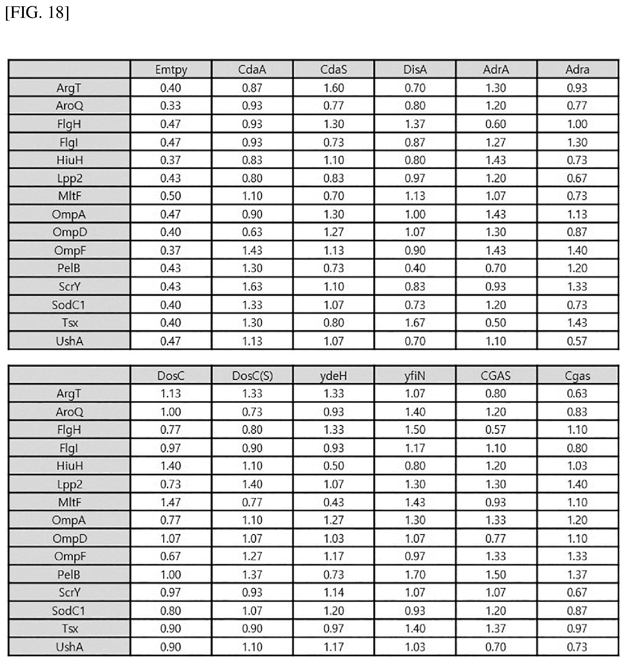

Since it was confirmed that antibiotic sensitivity increased when the STING agonist synthase was expressed inside the strain, the antibiotic sensitivity of recombinant strains into which a signal sequence was introduced was evaluated. First, when strains (SLppGpp) comprising a STING agonist synthase with a signal sequence were cultured in media treated with cefotaxime at various concentrations, it was confirmed, as in Example 4-2, that strains secreting the STING agonist synthase exhibited reduced antibiotic sensitivity (Fig. 18). When DNA constructs showing reduced antibiotic sensitivity were transformed into various strains using the SLppGpp strain and antibiotic sensitivity was evaluated, it was confirmed that introduction of the STING agonist synthase together with a signal sequence prevented lysis of the bacterial cells (Table 5).

**[Table 5]**

| | CdaA | PelB-DisA | Tsx-AdrA | UshA-Adra | MltF-ydeH | FlgH-CGAS |
|---|---|---|---|---|---|---|
| VNP2000 9 | 0.83 | 0.43 | 0.57 | 0.53 | 0.50 | 0.60 |
| SHJ2037 | 1.40 | 0.50 | 0.53 | 0.47 | 0.43 | 0.53 |
| CNC18 | 1.53 | 0.43 | 0.50 | 0.53 | 0.53 | 0.47 |
| DH5a | 0.73 | 0.57 | 0.53 | 0.47 | 0.43 | 0.53 |
| Nissle | 0.73 | 0.53 | 0.53 | 0.50 | 0.53 | 0.53 |

Subsequent experiments were performed using a CNC18 strain into which pJH18_PelB-DisA was introduced, which had been confirmed in the previous Examples to have no problems in protein secretion and strain growth, in order to reduce repetitive work.

### [4-3] Confirmation of immune-stimulating effects of transformed recombinant strains

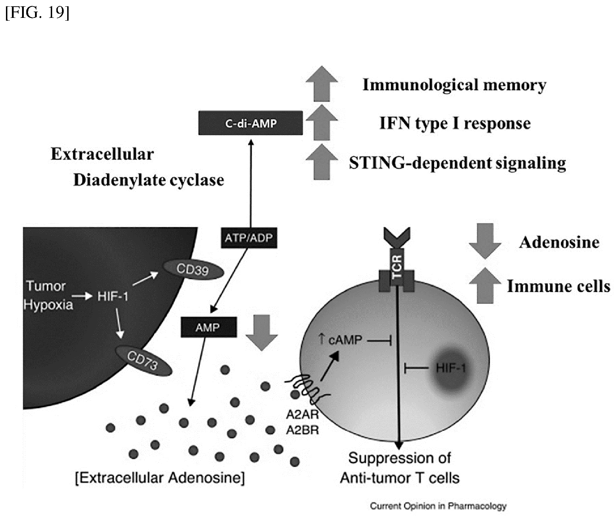

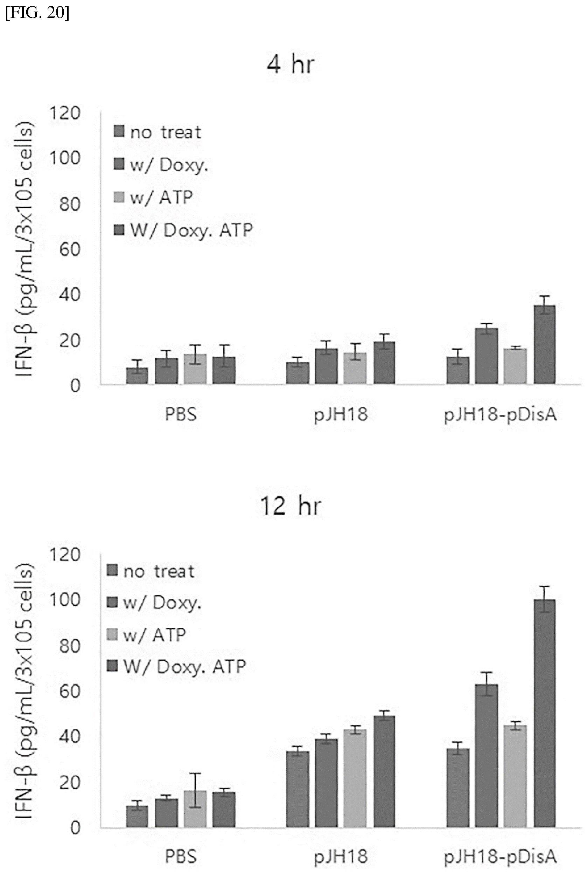

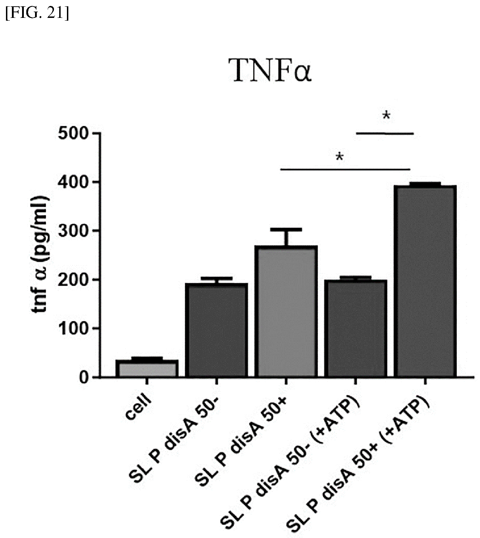

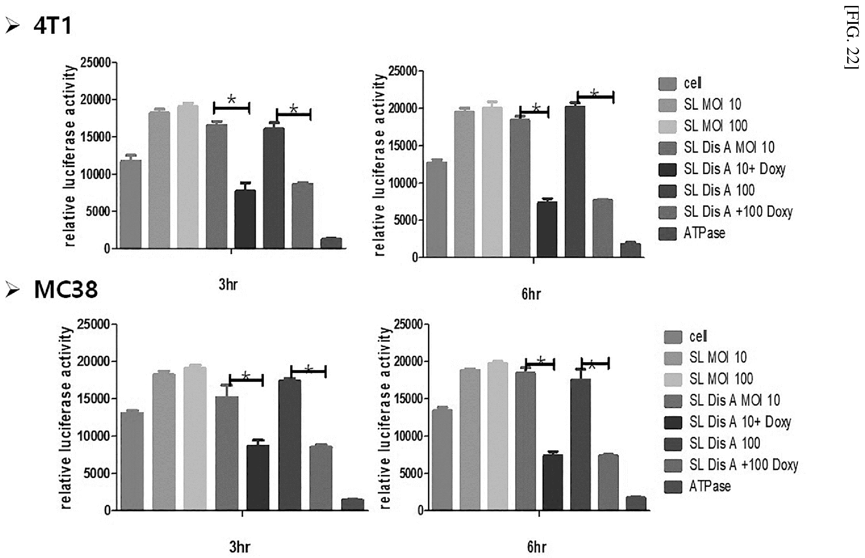

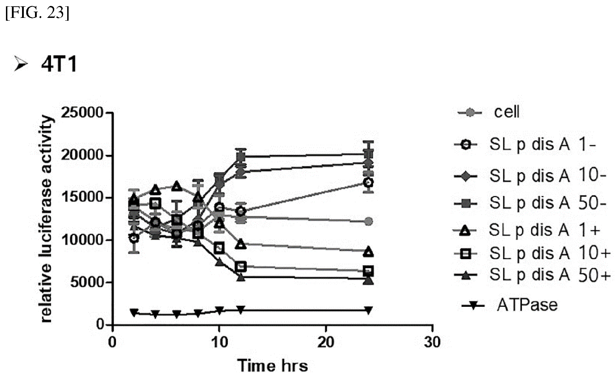

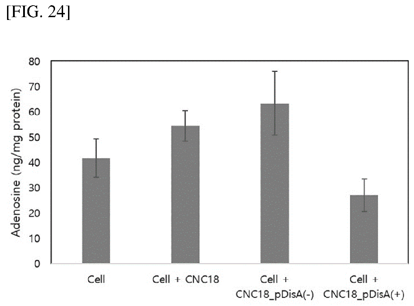

Since the strain transformed with the improved DNA construct secretes the STING agonist synthase extracellularly in a sustained manner, it can additionally utilize ATP present not only within the strain but also in the tumor microenvironment, thereby providing an additional synergistic effect of depleting tumor energy (Fig. 19). To verify this, RAW 264.7 cells (3×10⁵ cells/well) were treated with a *Salmonella* strain into which the PelB-DisA gene prepared in Example 4-1 was introduced, and additionally treated with 100 µM ATP and 200 ng/well doxycycline. As a result, as hypothesized, the pJH18_PelB-DisA experimental group treated with both ATP and doxycycline showed the most significantly increased immune-stimulating effect (Figs. 20 and 21). In addition, in order to confirm whether the transformed recombinant strain consumes ATP present in the tumor microenvironment, 4T1 and MC38 tumor cells were co-cultured with the strain, and ATP production in the medium was measured. As a result, it was confirmed that the recombinant strain treated together with doxycycline removed extracellular ATP (Figs. 22 and 23). The reduction of extracellular ATP may reduce the efficiency of tumor cells converting ATP into adenosine using CD39 and CD73 for immune evasion, thereby exposing the tumor cells to immune responses. To verify this, the medium obtained by co-culturing 4T1 cells with the recombinant strain as described above was analyzed using LC-MS to measure adenosine production, and as a result, it was confirmed that the amount of adenosine was lower in the pJH18_PelB-DisA experimental group treated together with doxycycline compared to the control group (Fig. 24). Through this, it was confirmed that the STING agonist synthase secreted outside the strain consumes extracellular ATP in the tumor microenvironment, thereby enhancing immune responses and reducing adenosine concentration, resulting in increased immune cell responses.

### [4-4] Confirmation of anticancer effects of transformed recombinant strains

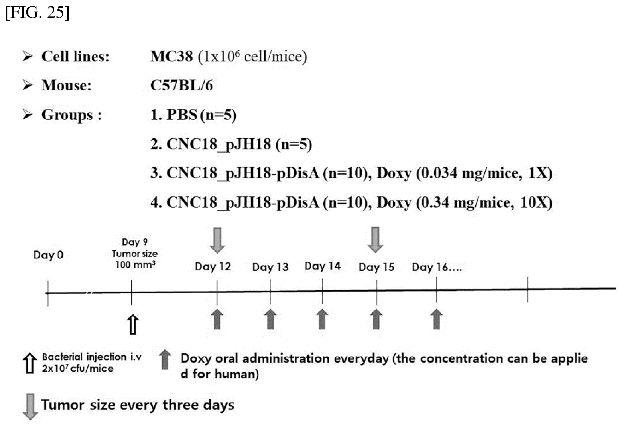

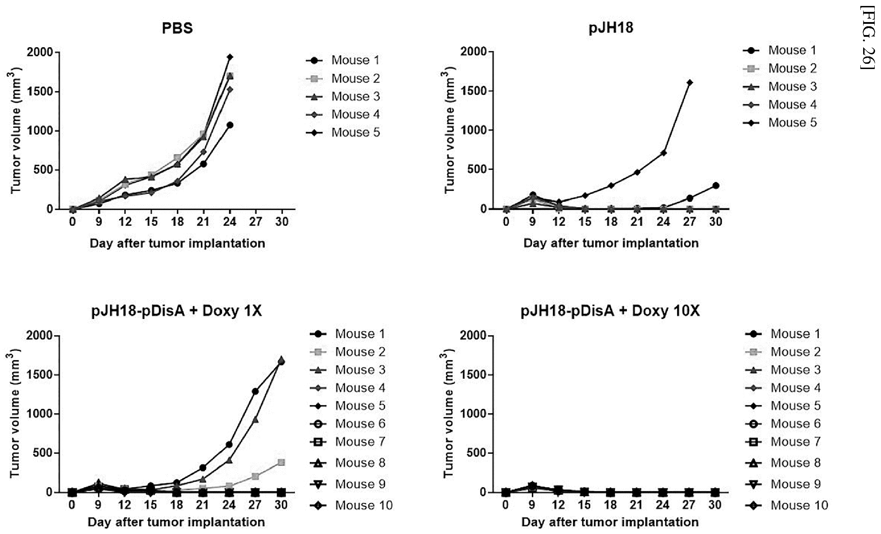

In order to confirm whether *Salmonella* strains transformed with the improved DNA construct expressing a STING agonist synthase induce anticancer effects in living animal subjects, tumor animal model experiments were performed while administering doxycycline. The overall in vivo experimental setup was the same as that of Example 2-3 (Fig. 25). As a result, it was confirmed that the pJH18_pDisA strain exhibited very excellent tumor suppression effects (Fig. 26).

### Example 5. Confirmation of combination therapy effects of an improved DNA construct expressing a STING agonist synthase

### [5-1] Confirmation of improved efficacy of combination with anticancer agents by transformed recombinant strains

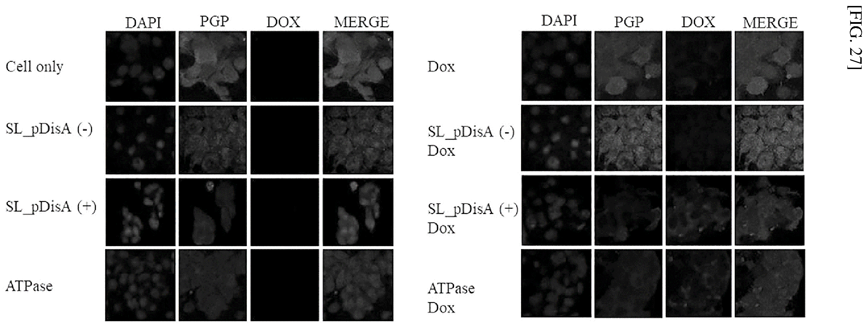

Tumor cells acquire resistance to anticancer agents by preventing intracellular accumulation using channels such as ABC transporters that export drugs out of the cell. At this time, ATP is used as an energy source, and reduction of ATP in the tumor microenvironment (extracellular ATP) decreases the energy source required to drive ABC transporters within cells, and due to functional impairment of channels such as decreased expression of P-gp, which is one of the ABC transporters, drugs cannot be properly exported and instead accumulate within cells, thereby inducing cell death. In order to confirm whether anticancer agents accumulate more within tumor cells due to a decrease in extracellular ATP, 4T1 cells were co-cultured with the recombinant strain to induce expression and secretion of the STING agonist synthase, followed by treatment with 1 µM doxorubicin. The treated cells were analyzed using confocal microscopy. As a result, tumor cells treated with the recombinant strain secreting the STING agonist synthase showed decreased P-gp expression and intracellular accumulation of doxorubicin, similar to the ATPase-treated group (Fig. 27). Through this, it was confirmed that the recombinant strain secreting the STING agonist synthase reduces extracellular ATP, thereby increasing intracellular accumulation of anticancer agents in tumor cells.

### [5-2] Confirmation of anticancer effects by combination of transformed recombinant strains and anticancer agents

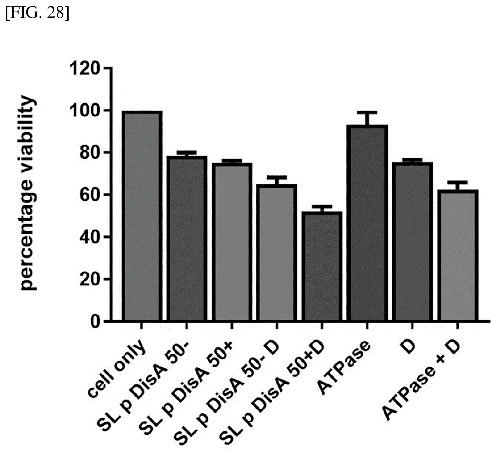

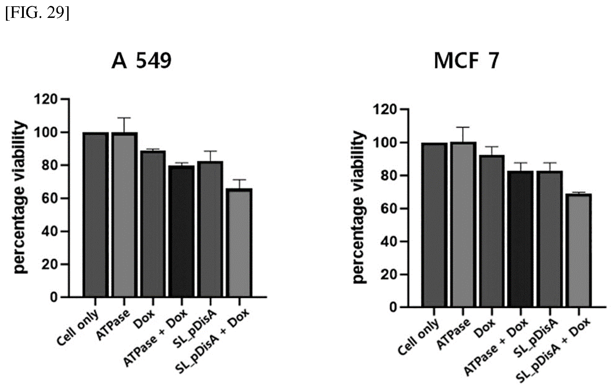

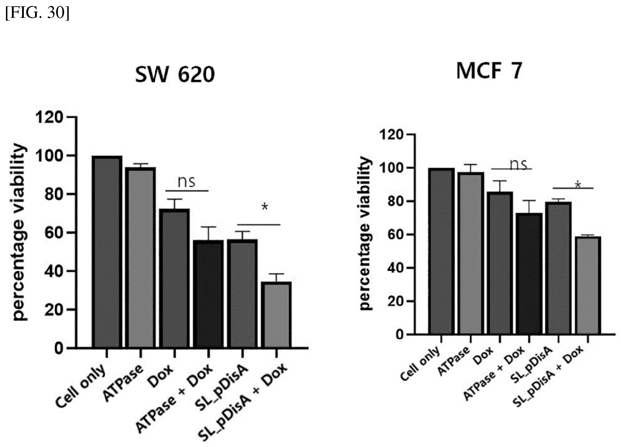

In order to confirm whether anticancer effects can be enhanced by co-administration of a recombinant strain secreting a STING agonist synthase and an anticancer agent, 4T1 cells were seeded in a 96-well plate at 3×10⁴ cells/well, and then treated with the recombinant strain secreting the STING agonist synthase and 1 µM doxorubicin, followed by measurement of cell viability using a CCK8 assay. As a result, compared to single-treatment groups, the group treated with both secretion of the STING agonist synthase and doxorubicin showed the lowest tumor cell viability (Fig. 28). In addition, when other tumor cells resistant to doxorubicin (A549 cells, MCF7 cells) were further examined in relation to intracellular accumulation of anticancer agents, the group treated with both secretion of the STING agonist synthase and doxorubicin showed the lowest tumor cell viability (Fig. 29). Furthermore, when tumor cells resistant to cisplatin (SW620 cells, MCF7 cells) were additionally examined, the group treated with both secretion of the STING agonist synthase and cisplatin showed the lowest tumor cell viability (Fig. 30). Through this, it was confirmed that co-administration of a recombinant strain secreting a STING agonist synthase and an anticancer agent can enhance anticancer effects.

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

### [Industrial Applicability]

The present invention relates to a DNA construct into which is introduced a gene for an enzyme that synthesizes a signaling substance for cancer treatment, e.g., a cyclic dinucleotide, or to a strain transformed with a vector comprising the DNA construct. The DNA construct or the strain transformed with a vector comprising the DNA construct, according to the present invention, targets cancer in a subject and then secretes the synthase in the surrounding environment of the cancer, and thus can very effectively prevent or treat cancer and, at the same time, can diagnose cancer in real time.

## Claims

1. A DNA construct comprising a gene encoding a cyclic dinucleotide synthase, and further comprising a secretion signal sequence.

2. The DNA construct of claim 1,
wherein the gene encoding the cyclic dinucleotide synthase is at least one selected from the group consisting of DisA, CdaA, CdaS, DosC, AdrA, ydeH, YfiN, CGAS, cdnC, relA, capV, cdnD, cdnE, cdnB, AKT1, gdpP, disA, dncV, cdnG, ZDHHC9, dacA, radA, STING3, argF, folP, cdaR, glmS, ybbP, dacA_2, disA_2, ossG, sle_31840, dacB, disA_1, disA_3, 1d0912, dacZ, cGlr1, cGlr2, guaA, cdnA, LYPLAL1, Rnf185, VC_A0931, VC_A0681, GG20550, GD11141, TcasGA2_TC003965, thsB1, eccD1, VC_A0210, CG7194, nadE, STING7, STING2, STING1, STING5, mucR, dgcP, pleD, dgcZ, dgcA, cdgI, dgcM, tpbB, csrA, dgcT, dgcN, dgcQ, dgcS, dgcF, dgcJ, dgcC, dgcE, dgcI, ydiV, vdcA, csrD, dosP, wspR, pdeF, csgD, tpbA, pdeR, and nbdA.

3. The DNA construct of claim 1,
wherein the secretion signal sequence comprises at least one leader sequence selected from the group consisting of pelB, ompA, ompB, ompC, ompD, ompE, ompF, ompT, phoA, TolB, TorT, LamB, LivK, TorA, SuflCT-B, LTIIb-B, BAP, MF α, SUC2, HasA, PspA, TlyA, OutD, YwbN, NprE, SpsA, LipA, LysK, XcpT, AmyE, EstA, BrkA, IcsA, CelA, ClyA, HlyA, VgrG, EspA, EsxA, AprE, FhuD, MglB, OppA, RbsB, Agp, FkpA, YtfQ, HdeA, HdeB, GlnH, LLO, and phoE.

4. The DNA construct of claim 1,
wherein expression of the gene encoding the cyclic dinucleotide synthase is regulated by a cis-acting element or a trans-acting element.

5. The DNA construct of claim 4,
wherein the cis-acting element is at least one selected from the group consisting of a ribosome binding site (RBS), a 5'-untranslated region (5'-UTR), a transcription factor binding site, and a terminator.

6. The DNA construct of claim 5,
wherein the transcription factor binding site is at least one selected from the group consisting of a promoter, an enhancer, and a silencer of the gene encoding the cyclic dinucleotide synthase.

7. The DNA construct of claim 4,
wherein the trans-acting element is at least one selected from the group consisting of a transcription factor, an aptamer, sRNA, and antisense RNA (asRNA).

8. The DNA construct of claim 1,
wherein the DNA construct further comprises at least one selected from the group consisting of a gene encoding an anticancer protein; a gene encoding a cytokine; a gene encoding a chemokine; a gene encoding an immune modulator; an oligonucleotide specific to a cancer antigen; and a gene encoding a reporter protein.

9. The DNA construct of claim 8,
wherein the anticancer protein is at least one selected from the group consisting of a toxin protein; an antibody specific to a cancer antigen or a fragment thereof; a tumor suppressor protein; an angiogenesis inhibitor; a cancer antigen; a prodrug-converting enzyme; a tumor microenvironment matrix-degrading enzyme; and a pro-apoptotic protein.

10. The DNA construct of claim 9,
wherein the toxin protein is at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA).

11. The DNA construct of claim 9,
wherein the tumor suppressor protein is at least one selected from the group consisting of Retinoblastoma protein (RB), p53 protein, Adenomatous polyposis coli (APC) protein, Phosphatase and tensin homologue (PTEN) protein, and cyclin dependent kinase inhibitor 2A (CDKN2A) protein.

12. The DNA construct of claim 9,
wherein the angiogenesis inhibitor is at least one selected from the group consisting of angiostatin, endostatin, thrombospondin, and a protease inhibitor protein.

13. The DNA construct of claim 9,
wherein the cancer antigen is at least one selected from the group consisting of α-fetoprotein (AFP), vascular endothelial growth factor receptor 2 (VEGFR2), survivin, legumain, and prostate cancer specific antigen (PCSA).

14. The DNA construct of claim 9,
wherein the prodrug-converting enzyme is at least one selected from the group consisting of thymidine kinase, cytosine deaminase, nitroreductase, purine nucleoside phosphorylase, carboxypeptidase G2, chromate reductase YieF, herpes simplex virus type I thymidine kinase/ganciclovir (HSV1-TK/GCV), and β-glucuronidase.

15. The DNA construct of claim 9,
wherein the tumor microenvironment matrix-degrading enzyme is at least one selected from the group consisting of collagenase, heparinase, hyaluronidase, protease, chymotrypsin, chymopapain, trypsin, caseinase, elastase, papain, deoxyribonuclease, matrix metalloproteinases (MMPs), cathepsins, β-glucuronidase, and serine proteases.

16. The DNA construct of claim 9,
wherein the pro-apoptotic protein is L-asparaginase (L-ASNase) or RNA-binding motif protein 5 (RBM5).

17. The DNA construct of claim 8,
wherein the oligonucleotide specific to a cancer antigen is a nucleotide sequence encoding at least one selected from the group consisting of an antisense oligonucleotide, an aptamer, siRNA, and shRNA.

18. The DNA construct of claim 8,
wherein the reporter protein is at least one selected from the group consisting of a fluorescent protein, luciferase, and a protein used for nuclear medicine or MRI imaging.

19. The DNA construct of claim 18,
wherein the fluorescent protein is at least one selected from the group consisting of green fluorescent protein (GFP), modified green fluorescent protein (MGFP), enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), and enhanced yellow fluorescent protein (EYFP).

20. The DNA construct of claim 18,
wherein the protein used for nuclear medicine or MRI imaging is at least one selected from the group consisting of herpes simplex virus thymidine kinase, a dopamine receptor, a somatostatin receptor, a sodium-iodide transporter, an iron receptor, a transferrin receptor, ferritin, and an iron transporter (magA).

21. A recombinant vector comprising the DNA construct of any one of claims 1 to 20.

22. A strain into which the recombinant vector of claim 21 is introduced.

23. The strain of claim 22,
wherein the strain is at least one selected from the group consisting of a *Salmonella* genus strain, a *Clostridium* genus strain, a *Bifidobacterium* genus strain, and an *Escherichia coli* genus strain.

24. A pharmaceutical composition for preventing or treating cancer, comprising the strain of claim 22 as an active ingredient.

25. The pharmaceutical composition of claim 24,
wherein the cancer is at least one selected from the group consisting of melanoma, fallopian tube cancer, brain cancer, small intestine cancer, esophageal cancer, lymphoma, gallbladder cancer, blood cancer, thyroid cancer, endocrine gland cancer, oral cancer, liver cancer, bile duct cancer, colorectal cancer, rectal cancer, cervical cancer, ovarian cancer, kidney cancer, gastric cancer, duodenal cancer, prostate cancer, breast cancer, brain tumor, lung cancer, anaplastic thyroid cancer, uterine cancer, colon cancer, bladder cancer, ureter cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma.

26. A composition for diagnosing cancer, comprising the strain of claim 22.

27. A method for providing information for diagnosing or treating cancer, comprising:
treating a biological sample isolated from a subject with the strain of claim 22.

28. The method of claim 27,
further comprising diagnosing cancer when a reporter protein is expressed from the strain.
